# EUROPEAN PATENT APPLICATION

(11) **EP 1 707 211 A1**
(43) Date of publication of application: **04.10.2006**
(21) Application number: 04819454.2
(22) Date of filing: 26.11.2004
(51) Int. Cl.: A61K 36/18, A61K 31/201, A61K 8/18, A61K 8/99, A61P 17/04, A61P 35/00, A61P 37/08, A61P 43/00, A23L 1/30, C07C 59/42

(54) **CERAMIDASE INHIBITOR**

(30) Priority: 28.11.2003 JP 2003400023
(71) Applicant: TAKARA BIO INC., Otsu-shi, Shiga 520-2193 (JP)
(72) Inventor: SANO, Mutsumi, Shiga 520-0014 (JP); IZU, Hiroyuki, Kutsatsu-shi Shiga 525-0025 (JP); IMAMURA, Mitsuo, Otsu-shi Shiga 520-2193 (JP); OKAMOTO, Motoko, Shizuoka 410-0044 (JP); KURITA, Toyohisa, Muko-shi Kyoto 617-0002 (JP); KATO, Ikunoshin, Shiga 529-1851 (JP)
(74) Representative: Vossius & Partner
(86) International application number: PCT/JP2004/017609
(87) International publication number: WO 2005/051406

(57) **Abstract**

The present invention provides a ceramidase activity inhibitor which inhibits ceramidase activity, specifically neutral/alkaline ceramidase activity, **characterized in that** the inhibitor comprises, as an active ingredient, a processed product derived from at least one plant selected from the group consisting of plants belonging to Ginkgoaceae, plants belonging to Cucurbitaceae, plants belonging to Rutaceae, plants belonging to Laminariaceae, plants belonging to Myrtaceae and plants belonging to Compositae, and a medicament, a quasi-drug, cosmetics and a food, each comprising the inhibitor.

## Description

### TECHNICAL FIELD

The present invention relates to a ceramidase activity inhibitor comprising a plant-derived processed product such as an extract, and a medicament, a quasi-drug, cosmetics and a food, each comprising the inhibitor.

### BACKGROUND ART

In recent years, sphingolipid (sphingoglycolipid, sphingomyelin and a metabolite thereof such as ceramide) has begun to attract attention rapidly as a signal molecule which regulates cellular growth, differentiation, apoptosis etc. In a disease such as cancer, autoimmune disease or infection, sphingolipid metabolism is significantly changed, thereby attracting strong interest as a target of drug design. In particular, ceramide is a lipid serving as a basic structure of sphingolipid and has been considered as an important factor which regulates life and death of a cell. At least three pathways for production of ceramide have been known at present. The three pathways are 1) *de novo* synthesis pathway beginning with condensation reaction of serine with palmitoyl CoA, 2) degradation system of sphingomyelin and 3) degradation system of glucosylceramide. Among these pathways, the degradation system of sphingomyelin is activated by a cell death-inducing cytokine such as TNF-α and Fas, serum depletion, irradiation with ultraviolet rays or radioactive rays, or oxidation stress. In addition, production of ceramide via degradation of sphingomyelin is also promoted by a differentiation factor such as vitamin D3, interferon-y or interleukin-1β. When a ceramide analogue C2-ceramide (D-erythro-N-acetylsphingosine) is exogenously added to a cell, phenomena such as apoptosis, differentiation induction and growth suppression are induced, or when a cell is treated with bacterial sphingomyelinase, ceramide is accumulated in the cell by degradation of sphingomyelin, and cellular growth suppression and apoptosis are induced in the same way as when C2-ceramide is added. Therefore, intracellular ceramide is considered to function as an important intracellular signal.

Ceramide is degraded by the action of ceramidase into a free fatty acid and a long-chain base sphingosine. Sphingosine is metabolized into sphingosine-1-phosphate through phosphorylation of a hydroxyl group at position 1. It has been known that sphingosine-1-phosphate, contrary to ceramide, exhibits a cellular growth promoting action and suppresses apoptosis induced by ceramide. As described above, in regulation of cellular growth, differentiation, cell death etc., the balance between intracellular ceramide and a metabolite thereof such as sphingosine or sphingosine-1-phosphate has been considered very important. Accordingly, a medicine which allows intracellular ceramide level in a cell to increase and allows sphingosine and sphingosine-1-phosphate level to lower is very useful as a medicine which regulates cellular growth, differentiation and cell death.

Ceramide is synthesized *de novo* from L-serine and palmitoyl-CoA, and as a substance which inhibits a palmitoyl-CoA: serinepalmitoyl transferase, which is a starting enzyme in this synthesis system, ISP-I (for example, Non-Patent Publication 1) and sphingofungin B (for example, NPP 2) have been known. As an inhibitor of acyl-CoA: sphinganine N-acyltransferase, which is an enzyme for converting dihydrosphingosine into dihydroceramide, fumonisin B1 (for example, NPP 3) has been known. Any of these substances have been known to inhibit *de novo* synthesis of ceramide, thereby reducing intracellular ceramide level.

As a substance which increases ceramide level, on the other hand, D-threo PDMP has been known as a substance which inhibits the activity of UDP-glucose: ceramide glucosyltransferase for transferring glucose to ceramide to synthesize glucosylceramide (for example, NPP 4), and this substance has been known to cause an increase in intracellular ceramide. However, this enzyme is a starting enzyme in biosynthesis of sphingoglycolipid, so that synthesis of sphingoglycolipid is also undesirably supperssed.

As a metabolic enzyme which regulates the balance between ceramide and sphingosine or sphingosine-1-phosphate, the importance of neutral/alkaline ceramidase (for example, NPPs 5, 6 and 7) and alkaline ceramidase (for example, NPP 8) have been remarked, and substances known to inhibit the activity of these enzymes are few. Only D-erythro-2-(N-myristoylamino)-1-phenyl-1-propanol (D-e-MAPP), a ceramide analogue, has been known to inhibit the activity of alkaline ceramidase to increase intracellular ceramide level (see, for example, Patent Publication 1 and NPP 9). However, the inhibitory activity of this substance on neutral/alkaline ceramidase is not strong and cannot be satisfactory. NPP 10 describes that another ceramide analogue, (1R,2R)-2-N-myristoylamino-1-(4-nitrophenyl)-1,3-propandiol (D-NMAPPD, or B13) efficiently inhibits acidic ceramidase rather than alkaline ceramidase, and there is no report on an effective inhibitor of neutral/alkaline ceramidase. NPP 9 describes that N-oleoylethanolamine has an inhibitory effect on acidic ceramidase, but this substance hardly exhibits an inhibitory effect on neutral/alkaline ceramidase. PP 2 discloses a ceramidase activity inhibitor comprising an extract of turmeric, strawberry geranium, Gotukola (*Centella asiantica*) or sea onion which suppresses degradation of ceramide in a horny layer of the skin, and a skin medicine for external application comprising these, but there is no description about the inhibitory effect of these plant extracts on neutral/alkaline ceramidase, and the effect of increasing ceramide level in the skin is not sufficient and cannot be satisfactory.

On the other hand, ceramide is a major intercellular lipid component in the skin and plays an important role in a moisture retention ability and barrier mechanism of the skin. In the skin horny layer of atopic dermatitis patients increasing in number in recent years, the ceramide content has been reduced, which has been considered as one of the causes of dry skin and abnormality in barrier function of a horny layer considered characteristic of atopic dermatitis. It has been recently reported that ceramidase-producing bacteria are detected more frequently in a patient with atopic dermatitis than in a normal individual (for example, NPP 11). Also, ceramidase-producing bacterium *Pseudomonas aeruginosa* strain AN17 is separated from a patient with atopic dermatitis, and its ceramidase is purified and cloned (for example, NPP 12 and 13). It is therefore considered that ceramidase produced by such bacteria degrades ceramide in the skin of a patient with atopic dermatitis, thereby decreasing ceramide in the horny layer, thereby lowering the barrier function of the skin and worsening the disease. However, an inhibitor which specifically inhibits the activity of microorganism-derived ceramidase has not been known at all.
Patent Publication 1: WO 97/44019
Patent Publication 2: JP-2002-308791 A
Non-Patent Publication 1: Miyake Y. and four others, Biochem. Biophys. Res. Commun., 211: 396-403 (1995)
Non-Patent Publication 2: Zweerink M. M. and four others, The Journal of Biological Chemistry, 267: 25032-25038 (1992)
Non-Patent Publication 3: Wang E. and four others, The Journal of Biological Chemistry, 266: 14486-14490 (1991)
Non-Patent Publication 4: Inokuchi J. and one other, J. Lipid Res., 28: 565-571 (1987)
Non-Patent Publication 5: Tani M. and five others, The Journal of Biological Chemistry, 275: 11229-11234 (2000)
Non-Patent Publication 6: Mitsutake S. and eight others, The Journal of Biological Chemistry, 276(26): 249-26, p. 259 (2001)
Non-Patent Publication 7: El Bawab S. and five others, The Journal of Biological Chemistry, 275: 21508-21513 (2000)
Non-Patent Publication 8: Mao C. and five others, The Journal of Biological Chemistry, 276: 26577-26588 (2001)
Non-Patent Publication 9: Bielawska A. and six others, The Journal of Biological Chemistry, 271: 12646-12654 (1996)
Non-Patent Publication 10: Raisova M. and nine others in FEBS Letters, 516: 47-52 (2002)
Non-Patent Publication 11: Ohnishi Y. and three others, Clin. Diagn. Lab. Immunol., 6: 101-104 (1999)
Non-Patent Publication 12: Okino N. and three others, The Journal of Biological Chemistry, 273: 14368-14373 (1998)
Non-Patent Publication 13: Okino N. and five others, The Journal of Biological Chemistry, 274: 36616-36622 (1999)

### DISCLOSURE OF THE INVENTION

### PROBLEMS TO BE SOLVED BY THE INVENTION

As described above, an effective inhibitor which inhibits the activity of neutral/alkaline ceramidase from an animal or a microorganism is not yet known and not industrially utilized at present. An object of the present invention is to provide a plant-derived inhibitor of ceramidase activity, particularly neutral/alkaline ceramidase activity, and a medicament, a quasi-drug, cosmetics and a food, each comprising the inhibitor.

### MEANS TO SOLVE THE PROBLEMS

In view of the circumstances described above, the present inventors made extensive study and as a result they considered that an inhibitor of neutral/alkaline ceramidase is effective for regulation of intracellular ceramide level, in its turn for cellular growth suppression, differentiation induction and regulation of apoptosis caused by intracellular ceramide. The present inventors have widely examined inhibitory activities of various substances on neutral/alkaline ceramidase, and as a result they have found that processed products derived from various plants and specific compounds derived from the processed products have specific inhibitory activities on neutral/alkaline ceramidase. The present inventors further have confirmed that these processed products derived from plants and the specific compounds derived from plants have activities of increasing the ceramide content in an animal cultured cell or a horny layer of the skin. On the basis of these findings, the present invention has been completed.

Specifically, the present invention provides a ceramidase activity inhibitor comprising, as an active ingredient, a processed product derived from a plant or a specific compound derived from a plant, such as an extract or an essential oil, for example, a steam distillate, a compressed product etc. derived from at least one plant selected from the group consisting of ginkgo (*Ginkgo biloba*), gourd, orange, gagome (*Kjellmaniella crassifolia* Miyabe), cucumber, grapefruit, wax gourd (*Benincasa cerifera* Savi), bitter cucumber (*Momordica charantia* L.), kelp (*Laminaria japonica* Areschoug), eucalyptus, mugwort *(Artemisia vulgaris* L. var *indica* Maxim.), lime and *wakame* seaweed (*Undaria pinnatifida*), and a regulator of intracellular and extracellular ceramide levels, a medicament such as an anti-inflammatory agent, an anticancer drug or a skin medicine for external application, a quasi-drug, cosmetics or a food, each comprising the inhibitor.

Specifically, a first invention of the present invention relates to a ceramidase activity inhibitor characterized in that the inhibitor comprises, as an active ingredient, a processed product derived from at least one plant selected from the group consisting of plants belonging to Ginkgoaceae, plants belonging to Cucurbitaceae, plants belonging to Rutaceae, plants belonging to Laminariaceae, plants belonging to Myrtaceae and plants belonging to Compositae. The plant in the first invention of the present invention is not particularly limited, but it is preferable that, for example, the plant belonging to Ginkgoaceae is ginkgo *(Ginkgo biloba,* Ginkoaceae); the plant belonging to Cucurbitaceae is at least one member selected from the group consisting of Oriental pickling melon (*Cucumis melo* L. var. *conomon* Makino), cucumber (*Cucumis sativus* L.), wax gourd (*Benincasa cerifera* Savi) and bitter cucumber (*Momordica charantia* L.); the plant belonging to Rutaceae is at least one member selected from the group consisting of orange *(Citrus sinensis, Citrus aurantium* or *Citrus reticulate*), grapefruit (*Citrus Paradisi*) and lime (*Citrus aurantifolia*); the plant belonging to Laminariaceae is at least one member selected from the group consisting of gagome (*Kjellmaniella crassifolia* Miyabe), kelp (*Laminaria japonica* Areschoug) and *wakame* seaweed (*Undaria pinnatifida*); the plant belonging to Myrtaceae is eucalyptus; and the plant belonging to Compositae is mugwort (*Artemisia vulgaris* L. var *indica* Maxim.).

A second invention of the present invention relates to a ceramide level regulator, characterized in that the regulator comprises the ceramidase activity inhibitor of the first invention.

A third invention of the present invention relates to a medicament characterized in that the medicament comprises the ceramidase activity inhibitor of the first invention. In the third invention of the present invention, the medicament may be a skin medicine for external application, a therapeutic agent or prophylactic agent for a disease requiring suppression of cellular growth, or a therapeutic agent or prophylactic agent for cancer.

A fourth invention of the present invention relates to a quasi-drug characterized in that the quasi-drug comprises the ceramidase activity inhibitor of the first invention.

A fifth invention of the present invention relates to cosmetics characterized in that the cosmetics comprise the ceramidase activity inhibitor of the first invention.

A sixth invention of the present invention relates to a food characterized in that the food comprises the ceramidase activity inhibitor of the first invention.

A seventh invention of the present invention relates to a compound having the following physicochemical properties, its derivative, or a pharmacologically acceptable salt thereof:
(1) Mass spectrum: m/z 565 (M+H)⁺;
(2) MS/MS analysis: when the above-mentioned (1) is a parent ion, the daughter ion is m/z 547, m/z 338;
(3) ¹H-NMR (deuterated chloroform): σ
   0.848, 0.860, 0.871, 1.236, 1.265, 1.277, 1.289, 1.354, 1.368, 1.507, 1.531, 1.584, 1.923, 1.934, 1.945, 1.964, 1.974, 2.018, 2.029, 2.041, 2.053, 2.208, 2.350, 2.462, 2.575, 2.957, 2.969, 3.728, 3.747, 3.770, 3.777, 3.783, 3.789, 3.971, 3.976, 3.989, 3.994, 5.321, 5.330, 5.346, 5.356, 5.368, 5.378, 5.394, 5.491, 5.501, 5.515, 5.527, 5.539, 5.605, 5.616, 5.629, 5.642, 5.653, 6.455, 6.468; and
(4) ¹³C-NMR (deuterated chloroform): σ 14.10, 22.68, 25.42, 29.20, 29.22, 29.34, 29.36, 29.49, 29.54, 29.62, 29.63, 29.65, 29.69, 31.91, 31.92, 32.42, 32.57, 32.60, 34.36, 40.56, 53.96, 62.45, 74.06, 122.17, 129.72, 130.88, 136.81, 171.83.

An eighth invention of the present invention relates to a ceramidase activity inhibitor characterized in that the inhibitor comprises, as an active ingredient, at least one member selected from the group consisting of the compound, the derivative and the salt of the seventh invention of the present invention.

A ninth invention of the present invention relates to a ceramide level regulator characterized in that the regulator comprises the ceramidase activity inhibitor of the eighth invention of the present invention.

A tenth invention of the present invention relates to a medicament characterized in that the medicament comprises the ceramidase activity inhibitor of the eighth invention of the present invention. In the tenth invention of the present invention, the medicament may be a skin medicine for external application, a therapeutic agent or prophylactic agent for a disease requiring suppression of cellular growth, or a therapeutic agent or prophylactic agent for cancer.

An eleventh invention of the present invention relates to a quasi-drug characterized in that the quasi-drug comprises the ceramidase activity inhibitor of the eighth invention of the present invention.

A twelfth invention of the present invention relates to cosmetics
characterized in that the cosmetics comprise the ceramidase activity inhibitor of the eighth invention of the present invention.

A thirteenth invention of the present invention relates to a food characterized in that the food comprises the ceramidase activity inhibitor of the eighth invention of the present invention.

### EFFECTS OF THE INVENTION

According to the present invention, there are provided a ceramidase activity inhibitor and a ceramide level regulator, a medicament, a quasi-drug, cosmetics and a food, each comprising the inhibitor.

The inhibitor of the present invention can be expected to exhibit an effect such as growth suppression, differentiation induction and apoptosis induction of an animal cell, and in its turn the medicine and the food useful in enhancement of health, each comprising the inhibitor of the present invention, can be expected to exhibit a therapeutic effect on a disease caused by abnormality in cellular growth or differentiation, such as inflammatory disease and malignant tumor. It can also be expected that a cream, a lotion or a bathing agent, containing the inhibitor of the present invention, is applied onto or contacted with the skin, thereby exhibiting an effect of improving the moisture retention and barrier function of the skin in a normal individual or a patient with atopic dermatitis by increasing the ceramide content in the skin horny layer. It can further be expected that an effect of ameliorating dry skin and even dermatitis by preventing reduction in ceramide in the skin in atopic dermatitis is brought about by inhibiting the activity of ceramidase produced by atopic dermatitis-associated microorganisms.

### BRIEF DESCRIPTION OF THE DRAWINGS

[Fig.1] A graph showing the effect of a ceramidase activity inhibitor of the present invention.
[Fig.2] Graphs each showing the effect of a ceramidase activity inhibitor of the present invention.
[Fig.3] A graph showing the effect of a ceramidase activity inhibitor of the present invention.
[Fig.4] A graph showing the effect of a ceramidase activity inhibitor of the present invention.
[Fig.5] A graph showing the effect of a ceramidase activity inhibitor of the present invention.
[Fig.6] A profile showing a mass spectrum of a compound of the present invention.
[Fig.7] A profile showing a mass spectrum of a compound of the present invention in MS/MS analysis.
[Fig.8] A profile showing a ¹H-NMR spectrum of a compound of the present invention.
[Fig.9] A profile showing a ¹³C-NMR spectrum of a compound of the present invention.

### BEST MODE FOR CARRYING OUT THE INVENTION

(1) Ceramidase Activity Inhibitor of the Present Invention
The ceramidase activity inhibitor of the present invention is a substance which inhibits the enzyme activity of ceramidase, but does not necessarily act directly on ceramidase to inhibit the activity. The ceramidase activity inhibitor of the present invention includes, for example, a ceramidase inhibitor. For the sake of convenience, the ceramidase activity inhibitor may be referred to hereinafter as a ceramidase inhibitor in some cases.

The inhibitory action of the ceramidase activity inhibitor of the present invention on the enzyme activity of ceramidase refers to an action of reducing the activity of ceramidase as compared with the inherent activity of ceramidase, and can be confirmed, for example, according to a method described in Reference Example 1 described later. The inhibitory action on the enzyme activity of ceramidase is not particularly limited as long as the enzyme activity is reduced as compared with the inherent activity of ceramidase, and it is preferable that the activity is inhibited, for example, by 5%, preferably 10%, more preferably 20%, even more preferably 40%, 60%, 80%, and 90%.

The ceramidase inhibitor of the present invention comprises, as an active ingredient, a processed product derived from a predetermined plant or a specific compound etc. derived from the processed product, and exhibits an excellent inhibitory activity on ceramidase, particularly on neutral/alkaline ceramidase. The ceramidase inhibitor of the present invention may be composed of the active ingredient itself. The phrase "compound etc. derived from the processed product" merely indicates the origin of the compound etc., and a compound etc. which are the same as "the compound etc." and are separately synthesized irrelevantly to the plant are also encompassed in the active ingredient of the present invention.

The plant-derived processed product used as the active ingredient in the present invention (hereinafter referred to sometimes as a processed product of the present invention) is not particularly limited as long as the processed product is obtained by subjecting a plant to artificial treatment, and exemplified by an extract and essential oil (for example, steam distillate, squeezed fluid, compressed fluid, solvent extract and supercritical fluid extract). These processed products can be used alone or as a mixture of two or more kinds. The plant used in the present invention includes at least one member selected from the group consisting of plants belonging to Ginkgoaceae, plants belonging to Cucurbitaceae, plants belonging to Rutaceae, plants belonging to Laminariaceae, plants belonging to Myrtaceae and plants belonging to Compositae.

The processed product derived from these plants is not particularly limited as long as the processed product exhibits an inhibitory activity on at least neutral/alkaline ceramidase. As the plant, the following exemplary plants can be preferably used because their processed products are excellent in inhibitory action on ceramidase activity.

For example, as the plant belonging to Ginkgoaceae used in the present invention, ginkgo (*Ginkgo biloba,* Ginkoaceae) can be preferably used, and leaves thereof are mainly used.

As the plant belonging to Cucurbitaceae used in the present invention, Oriental pickling melon *(Cucumis melo* L. var. *conomon* Makino), Oriental melon (*Cucumis melo* L. var. *makuwa* Makino) and muskmelon (*Cucumis melo* L.) can be preferably used, and fruits thereof are mainly used. Further, cucumber (*Cucumis sativus* L.), wax gourd (*Benincasa cerifera* Savi) and bitter cucumber (*Momordica charantia* L.) can also be preferably used, and fruits thereof are mainly used. As the plant belonging to Cucurbitaceae, it is more preferable to use one or more members selected from Oriental pickling melon, cucumber, wax gourd and bitter cucumber.

As the plant belonging to Rutaceae used in the present invention, it is preferable to use one or more members selected from orange *(Citrus sinensis, Citrus aurantium* or *Citrus reticulate),* grapefruit *(Citrus Paradisi)* or lime *(Citrus aurantifolia*), and their fruits are mainly used. Limonene, which is an oil extracted from orange peel, can also be used in the present invention.

As the plant belonging to Laminariaceae used in the present invention, particularly as a plant belonging to Laminariaceae of Phaeophycaeae, it is preferable to use one or more members selected from gagome (*Kjellmaniella crassifolia* Miyabe), kelp (*Laminaria japonica* Areschoug) or *wakame* seaweed (*Undaria pinnatifida*), and leaves, stems or sporophylls thereof are mainly used.

The plant belonging to Myrtaceae used in the present invention, eucalyptus, that is, an evergreen eucalyptus tree or a closely related plant thereof, *Eucalyptus globulus, Eucalyptus citriodora* or *Eucalyptus dives,* can be preferably used, and leaves thereof are mainly used.

As the plant belonging to Compositae used in the present invention, mugwort (*Artemisia vulgaris* L. var. *indica* Maxim) can be preferably used, and leaves thereof are mainly used.

When the plants described above are used, the tissues of the plants used in the present invention are not limited to those mentioned above; for example, rhizomes, leaves, fruits, sporophylls or the whole of the plant body can be used. A processed product such as a fragmented product, a powdered product and a dried product can be used as a raw material for the processed product of the present invention. To prepare the ceramidase activity inhibitor of the present invention, the plants described above can be used alone or in combination thereof.

These plants used in the present invention, processed products thereof, and the specific compounds of the present invention described later are not known to have an inhibitory effect on neutral/alkaline ceramidase at all.

In a method of extracting the plant extract used in the present invention, the plant extract can be obtained, for example, by subjecting the above-mentioned plant as it is, or dried and milled product thereof, to extraction with a solvent used ordinarily in extraction of a plant component and then distilling the solvent off. The extraction solvent includes, for example, lower alcohols such as methanol, ethanol, propanol, isopropyl alcohol, butanol and isobutyl alcohol, or polyhydric alcohols such as ethylene glycol, propylene glycol and 1,3-butylene glycol, various organic solvents such as acetone, ethyl acetate, ether, chloroform, dichloroethane, toluene, n-hexane and petroleum ether, or water, and these solvents can be used alone or in combination thereof. Particularly, water, ethanol, propylene glycol, 1,3-butylene glycol, or an aqueous solution of ethanol, an aqueous solution of propylene glycol, and an aqueous solution of 1,3-butylene glycol are preferable from the viewpoint of extraction efficiency and yield of an extract.

In the extraction method, conditions usually used in extraction of a plant component can be used, and for example, the plant may be immersed in or thermally refluxed with an extraction solvent at 4° to 100°C for several hours to several weeks, and the best method can be properly established depending on the plant used as the starting material, the site and form of the plant to be used. In this case, the inhibitory activity of the extract on a neutral/alkaline ceramidase is determined according to a method described later in Reference Example 1, and the extraction method may be established such that inhibitory activity is attained at its maximum.

When an essential oil is used as the processed product of the present invention, the essential oil can be prepared in a method conventionally employed. For example, the essential oil can be prepared from the above-mentioned plant by a steam distillation method, a squeeze method, a compression method, a solvent extraction method or a supercritical fluid extraction method etc.

The method of extracting a plant extract or the method of preparing an essential oil may be carried out in accordance with a known method, and reference may be made to, for example, Bioseparation Handbook edited in 1996 by the specific study group of Bioseparation Engineering, the Society of Chemical Engineers, Japan.

The plant extract or essential oil described above can be used directly as the processed product of the present invention or may further be purified by chromatography such as adsorption partition chromatography, gel filtration chromatography, ion exchange chromatography, normal phase partition chromatography and reverse phase partition chromatography, and the inhibitory activity of the fractionated fractions on a neutral/alkaline ceramidase is determined, whereby a highly active fraction can be obtained with high purity, or a substance having an inhibitory activity on a neutral/alkaline ceramidase can be isolated and used. A fraction having high inhibitory activity on neutral/alkaline ceramidase may be used alone, or as a mixture of a plurality of fractions.

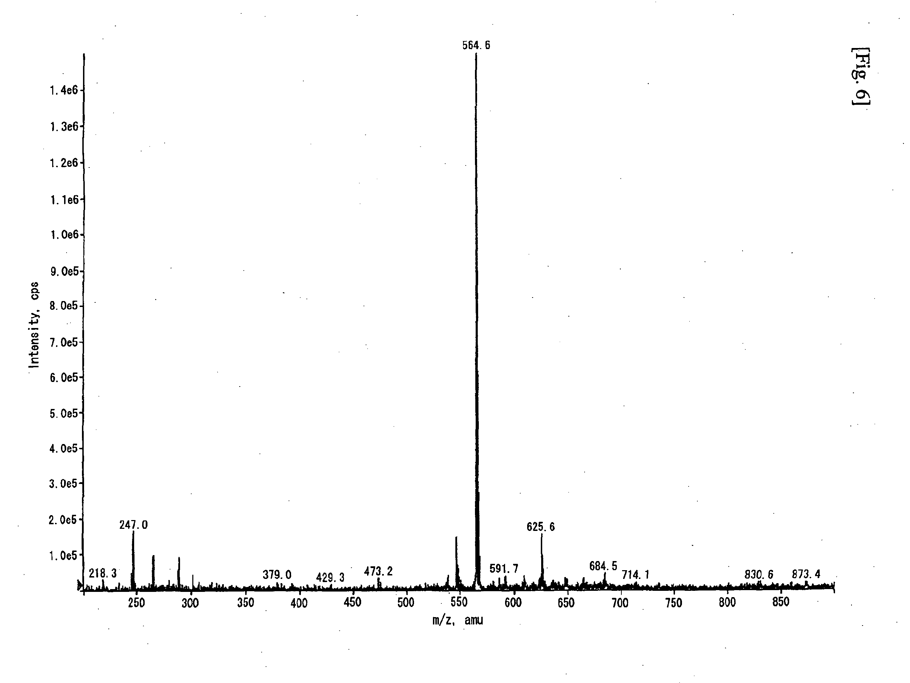

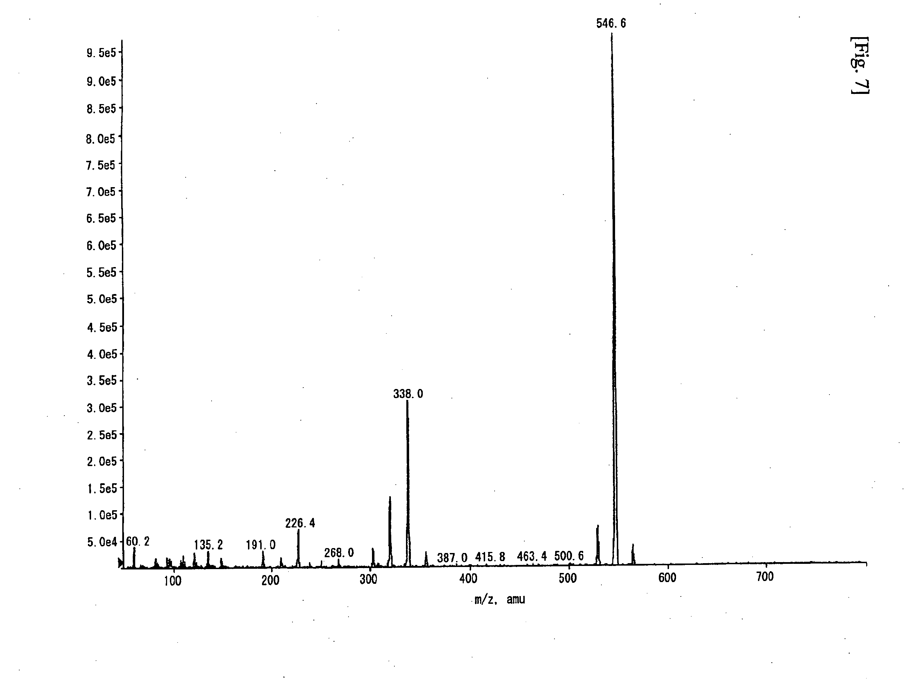

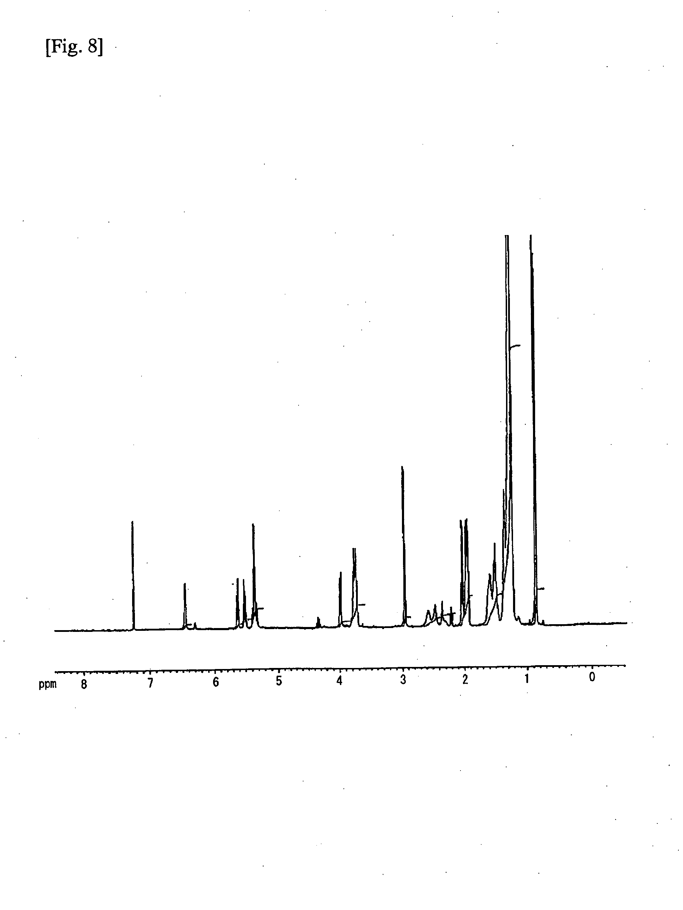

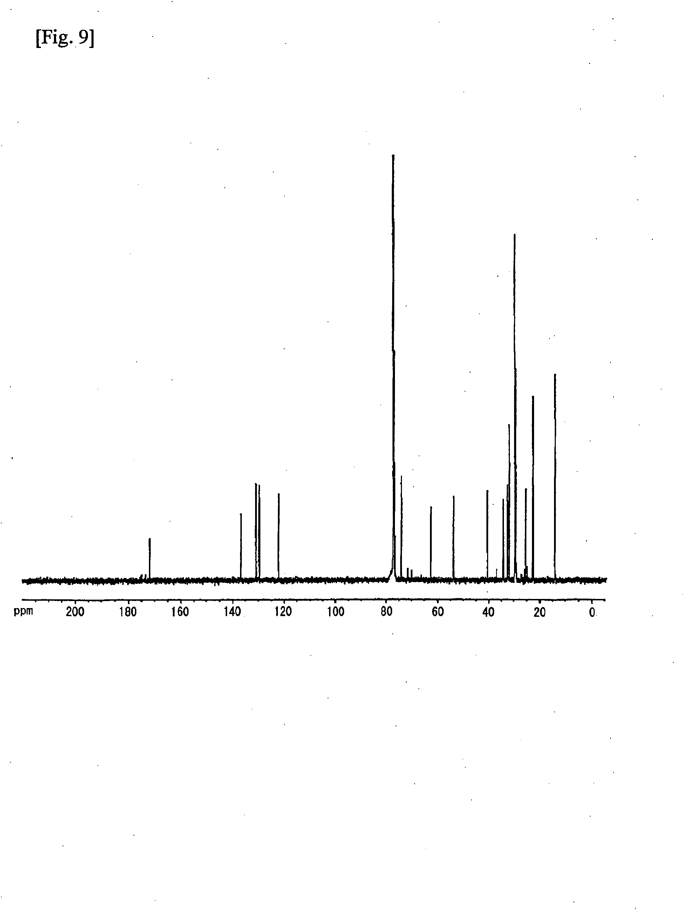

As the ceramidase activity inhibitor of the present invention, at least one member selected from the group consisting of the following compound, its derivative and a pharmacologically acceptable salt thereof can be used as the active ingredient. The compound is a novel compound obtained from a sporophyll of *wakame* seaweed (*Undaria pinnatifida*) having an inhibitory action on a ceramidase activity, and its mass spectrum is shown in Fig. 6, its MS/MS mass spectrum in Fig. 7, its ¹H-NMR spectrum in Fig. 8, and its ¹³C-NMR spectrum in Fig. 9, respectively. For details of the method of isolating the compound, the method of determining various spectra/determination results, the inhibitory action on ceramidase activity and the like, see Example 11 described later. That is, the compound having the above-mentioned physicochemical properties, its derivative and a pharmacologically acceptable salt thereof (also referred to herein as the compound etc. in some cases) have an inhibitory action on ceramidase activity and can exhibit an equivalent function to that of the processed product of the present invention. The compound etc. is isolated for the first time in the present invention and encompassed by the present invention.

The compound of the present invention is exemplified by the compound represented by the following formula (1):

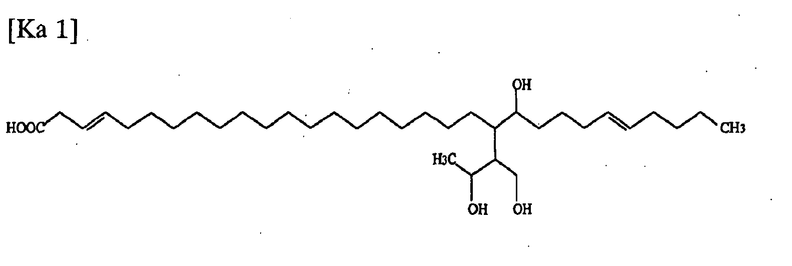

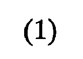

In the present invention, the salt is preferably a pharmacologically acceptable salt. The derivative of the compound of the above-mentioned formula (1) used in the present invention includes, for example, a derivative (prodrug) such as an ester, which is easily hydrolyzed in the living body to exhibit the desired effect. The derivative of the present invention also encompasses a derivative formed by administration of the compound of the present invention to a mammal and metabolism of the compound. The prodrug may be prepared in accordance with a known method. The derivative may also be a salt thereof. Accordingly, the active ingredient of the present invention includes a derivative of the compound of the present invention and a salt thereof as long as the desired effect of the present invention can be obtained. Various isomers of the compound used in the present invention, such as an optical isomer, a keto-enol tautomer and a geometrical isomer, and an isolate of each isomer, can be all used in the present invention as long as they have an inhibitory action on ceramidase activity.

The salt used in the present invention is exemplified by, for example, an alkali metal salt, an alkaline earth metal salt, a salt of an organic base etc. The salt is preferably a pharmacologically acceptable salt. The pharmacologically acceptable salt used in the present invention refers to a salt of the compound which is substantially atoxic to an organism and which has an inhibitory action on ceramidase activity. The salt includes, for example, a salt with sodium, potassium, calcium, magnesium, ammonium, or protonated benzathine (N,N'-di-benzylethylenediamine), choline, ethanolamine, diethanolamine, ethylenediamine, meglamine (N-methylglucamine), benethamine (N-benzylphenetylamine), piperazine, tolomethamine (2-amino-2-hydroxymethyl-1,3-propanediol), or the like.

The preparation form of the ceramidase inhibitor of the present invention is not particularly limited, as long as it inhibits ceramidase activity. An additive used for the above-mentioned ceramidase activity inhibitor is not particularly limited, and a known base material can be used. The ceramidase inhibitor of the present invention can be prepared, for example, in the form of a reagent by properly mixing the active ingredient of the present invention with a known base material. The ceramidase inhibitor of the present invention can also be compounded into a material used, for example, as a ceramide level regulator, a medicament including a skin medicine for external application, a cellular growth suppressor, a therapeutic agent or prophylactic agent for cancer, a quasi-drug, cosmetics or a food.

The content of the active ingredient in the ceramidase inhibitor of the present invention is, when the active ingredient is the processed product of the present invention, preferably 0.000001 to 100% by weight, more preferably 0.00001 to 20% by weight, or when the active ingredient is the compound etc. of the present invention, preferably 0.000001 to 100% by weight, more preferably 0.00001 to 20% by weight, on a dry-weight basis. When the ceramidase inhibitor of the present invention is incorporated into the above-mentioned medicament etc., the amount is not particularly limited, but is preferably in the range of about 0.000001 to 100% by weight, more preferably about 0.00001 to 20% by weight, even more preferably about 0.0001 to 10% by weight, on a dry-weight basis.

By the physiological action of the active ingredient of the present invention, the ceramidase activity inhibitor comprising the active ingredient is also useful for study on cellular growth, cancer, inflammation, wrinkle formation (mechanism of reduction in skin elasticity and of skin thickening), barrier function of horny layer of the skin, dry skin, microbial infection and skin immunity or for screening of a medicament for a disease associated with sphingolipid (ceramide), a skin medicine for external application, a cellular growth suppressor and a therapeutic agent or prophylactic agent for cancer, in addition to the medicament of the present invention.

The term "comprising" herein is sometimes used in meaning encompassing dilution and addition. The "addition" encompasses an embodiment in which the active ingredient used in the present invention is added to a starting material, and the "dilution" encompasses an embodiment in which the active ingredient used in the present invention is added to a starting material or a starting material is added to the active ingredient used in the present invention, whereby the active ingredient is diluted.

(2) Ceramide Level Regulator of the Present Invention
The ceramide level regulator of the present invention is not particularly limited as long as it has an effect of regulating ceramide level by the ceramidase-inhibiting action of the ceramidase inhibitor of the present invention. As used herein, the "effect of regulating ceramide level" refers to an effect achieved by the increase, by the ceramidase inhibitor, of ceramide level in cultured cells or in an artificial skin or the reduction, by properly selecting the amount of the ceramidase inhibitor added, depending on increased ceramide level, to desired ceramide level, as shown in Examples 6 to 10 described later. The ceramide level regulator of the present invention regulates the amount of ceramide serving as a basic structure of sphingolipid, and can thus regulate the amount of sphingolipid serving as an information molecule which regulates cellular growth, differentiation, apoptosis etc., or the amount of ceramide serving as a major intercellular lipid component involved in the moisture retention and barrier function of the skin. Further, the ceramide level regulator of the present invention functions not only as a regulator of ceramide level but also as a regulator of sphingosine level, fatty acid level and sphingosine-1-phosphate level by being involved in ceramide metabolism, so that the ceramide level regulator can be used as a sphingosine level regulator, a fatty acid level regulator or a sphingosine-1-phosphate level regulator.

The preparation form of the ceramide level regulator of the present invention is not particularly limited and can be prepared, for example, in the form of a reagent by properly mixing the ceramidase inhibitor of the present invention with a known base material. The content of the ceramidase inhibitor (active ingredient) of the present invention in the ceramide level regulator of the present invention is in the same range as for the amount of the ceramidase inhibitor of the present invention in the case where the ceramidase inhibitor is compounded into the medicament etc. Usage of the ceramide level regulator of the present invention is not limited particularly, and usage as a reagent for biochemical study on ceramide is preferable.

(3) Medicament etc. of the Present Invention The medicament of the present invention comprises the ceramidase activity inhibitor of the present invention and is provided as a skin medicine for external application (a moisturizing agent, a barrier-function keeping agent, a skin elasticity-improving or keeping agent, a skin-thickening ameliorative or prophylactic agent, and preparations for these applications, such as an ointment, cream, milky lotion, lotion, pack, and bathing agent), a cellular growth suppressor, an anti-inflammatory agent, a therapeutic agent or prophylactic agent for cancer, and a therapeutic agent or prophylactic agent for a disease associated with signal transduction mediated by ceramide or a metabolite thereof. In particular, the medicament of the present invention is very useful as a skin medicine for external application, a therapeutic agent or prophylactic agent for a disease requiring suppression of cellular growth, or a therapeutic agent or prophylactic agent for cancer.

Ceramide is a major intercellular lipid component in the skin, and plays an important role in the moisture retention and barrier mechanism of the skin. In a horny layer of the skin of a patient with atopic dermatitis, the ceramide content is reduced, and this reduction is considered as one of the causes for dry skin and abnormality in a barrier function in the horny layer, characteristic of atopic dermatitis, which might result in worsening of the disease. It has been also known that the reduction in ceramide level leads to reduction in skin elasticity and promotion of skin thickening. According to the active ingredient of the present invention, intracellular and extracellular ceramide levels can be increased or can be prevented from being reduced, through inhibition of ceramidase activity which degrades ceramide, as shown in the Examples described later. Accordingly, the medicament comprising the active ingredient of the present invention can be expected to have a therapeutic or prophylactic effect on the disease or symptom described above.

Sphingolipids (sphingoglycolipid, sphingomyelin and a metabolite thereof such as ceramide, sphingosine or sphingosine-1-phosphate) associated with regulation of cellular growth, differentiation, apoptosis etc. have been remarked with respect to metabolism in a disease such as cancer, autoimmune disease, infection, and inflammation such as dermatitis and arthritis and signals mediated via these molecules and signal transduction. In particular, ceramide is a lipid serving as a basic structure of sphingolipid and is considered as an important factor which regulates life and death of a cell. Accordingly, abnormality in ceramide metabolism can be a cause of abnormality in cellular growth or differentiation, thus causing a disease such as an inflammatory disease and malignant tumor (cancer) caused by the above-mentioned abnormality. For example, it has been known that when a cancer cell is treated with an anticancer agent or irradiated with radioactive rays, intracellular ceramide level is increased, to cause the cancer cell to undergo apoptosis. It has been reported that in a cancer cell which acquired anticancer drug resistance, the activity of a ceramide-metabolizing enzyme such as glucosyltransferase, which transfers glucose to ceramide, is increased, thereby preventing intracellular ceramide level from increasing (for example, Lavie, Y., Cao, H., Bursten, S.L., Giuliano, A.E., and Cabot, M.C., J. Biol. Chem. 271: 19530-19536 (1996)). According to the active ingredient of the present invention, cellular growth suppression or apoptosis induction can be effected through inhibition of the activity of ceramidase which degrades ceramide, as shown in the Examples described later. Accordingly, it can be expected that the medicament of the present invention comprising the active ingredient of the present invention suppresses occurrence of abnormality in ceramide metabolism or ameliorates the abnormality to show a therapeutic or prophylactic effect for the disease or symptom described above. Since the medicament of the present invention can exhibit a suppressive effect on cellular growth, the medicament is usable as a cellular growth suppressor.

As described above, since glucosyltransferase may be involved in the abnormality of ceramide metabolism in some cases, the medicament of the present invention can be expected to have a therapeutic or prophylactic effect on cancer endowed with, for example, multidrug resistance by use, for example, together with an inhibitor of glucosyltransferase as a ceramide-metabolizing enzyme other than ceramidase thereby exhibiting.

Subsequently, the method of producing the medicament of the present invention is described. The medicament of the present invention comprises the ceramidase activity inhibitor of the present invention as an active ingredient, and the active ingredient may be combined with a known pharmaceutical carrier, to prepare into a formulation. Generally, the active ingredient of the present invention is compounded with a pharmacologically acceptable liquid or solid carrier, and a solvent, a dispersant, an emulsifier, a buffer, a stabilizer, an excipient, a binder, a disintegrant, a lubricant or the like is added if desired, to form a solid preparation such as a tablet, a granule, a powder, a fine powder and a capsule or a liquid agent such as a common liquid agent, a suspension agent and an emulsion agent. The medicament of the present invention can also be a dried product that can be converted into liquid by adding an appropriate carrier before use, or can be a preparation for external application.

The pharmaceutical carrier can be selected depending on the administration route and administration form of the medicament of the present invention. In the case of the oral preparation, for example, starch, lactose, saccharose, mannitol, carboxymethyl cellulose, corn starch, an inorganic salt etc. can be used. During the preparation of the oral preparation, a binder, a disintegrant, a surfactant, a lubricant, a fluidity accelerator, a corrective, a coloring agent, a flavor or the like can be further compounded.

The parenteral preparation, on the other hand, can be prepared in accordance with a usual manner by dissolving or suspending the active ingredient of the present invention in a diluent such as distilled water for injection, physiological saline, an aqueous solution of glucose, vegetable oil for injection, sesame oil, peanut oil, soybean oil, corn oil, propylene glycol, or polyethylene glycol, and, if desired, by adding a microbicide, a stabilizer, an osmotic regulator, a soothing agent or the like.

The skin medicine for external application includes a solid, semisolid or liquid preparation for transdermal administration. The skin medicine also includes a suppository etc. The skin medicine can be formed, for example, as an emulsion such as a milky lotion and a lotion, a liquid preparation such as a tincture for external application, an ointment such as an oily ointment and a hydrophilic ointment, and a patch for transdermal administration, such as a film, a tape and a poultice.

The cellular growth suppressor and the therapeutic agent or prophylactic agent for cancer encompassed by the present invention can be expected to exhibit the apoptotic induction action, cellular growth suppression or prophylactic effect and cancer therapeutic effect of the ceramidase activity inhibitor of the present invention. The medicament of the present invention can be produced properly by a method known in the pharmaceutical field. The content of the ceramidase inhibitor (active ingredient) of the present invention in the medicament of the present invention can be exemplified by the same range as for the amount of the ceramidase inhibitor of the present invention in the case where the ceramidase inhibitor is compounded into the medicament etc.

The medicament of the present invention can be administered via an appropriate administration route depending on the form of the preparation. The administration method is not particularly limited and the preparation can be administered internally, externally or through injection. The injection can be administered, for example, intravenously, intramuscularly, subcutaneously, intradermally etc.

The dose of the medicament of the present invention can be properly determined and varies depending on the form of the preparation, administration method, intended use and the age, weight and symptoms of the patient to be administered with the medicament. Generally, the dose of the active ingredient comprised in the preparation, on a dry-weight basis, is preferably 0.1 to 2000 mg/ kg (body weight) per day per adult. As a matter of course, the dose varies depending on various conditions, and thus a dose lower than the above-mentioned range may be sufficient, or a dose higher than the above-mentioned range may be necessary. The medicament may be administered once or in divided portions several times per day in an amount in the desired range. The medicament of the present invention can be orally administered as it is, or can be ingested daily by adding it into any food or beverage.

When the medicament of the present invention is used as a skin medicine for external application, the preparation may be properly compounded not only with the active ingredient of the present invention but also with an ingredient usually used in a skin medicine for external application such as cosmetics and a medicament, for example, an aqueous component, an oily component, a powdery component, alcohol, a moisturizing agent, a thickener, a UV absorber, a whitener; a preservative, an antioxidant, a surfactant, a flavor and a colorant, if needed.

The quasi-drug of the present invention is characterized in that the quasi-drug comprises the ceramidase activity inhibitor of the present invention. The quasi-drug of the present invention is not particularly limited, and includes a preparation acting gently on a human body, such as a collutorium, a dentifrice, a mouth wash, a stomachic refresher, a vitamin-comprising health agent, a troche, a sunscreen lotion, soap, a hair dye, a sanitary napkin, a bathing agent, a preparation directed to prevent mouth odor, body odor, heat rash and alopecia, and a preparation directed to restore or remove hair, and a preparation corresponding to these. The quasi-drug of the present invention can be expected to have the same effect as that of the medicament of the present invention.

The quasi-drug of the present invention can be prepared in any form by properly mixing the ceramidase inhibitor of the present invention with a known base. The content of the ceramidase inhibitor (active ingredient) of the present invention in the quasi-drug of the present invention is in the same range as for the content of the inhibitor in the case where the ceramidase inhibitor of the present invention is compounded into the medicament etc.

(4) Cosmetics of the Present Invention The cosmetics of the present invention comprise the ceramidase activity inhibitor of the present invention, and the desired effect of the cosmetics of the present invention is exhibited based on the above-mentioned physiological action possessed by the inhibitor (active ingredient) contained in the cosmetics. It is estimated that moistening properties, barrier effect, the tautness and elasticity of the skin, and moisture retention (moisture) can be improved, if the amount of ceramide in the horny layer is increased and/or inside the skin including the horny layer. Therefore, according to the cosmetics of the present invention, for example, the tautness and elasticity of the skin can be sufficiently improved. That is, there are provided cosmetics comprising the ceramidase activity inhibitor of the present invention as an active ingredient, wherein the cosmetics are excellent in an action of improving moisture retention or an action of preventing deterioration of moisture retention, an action of improving the barrier effect or an action of preventing deterioration of the barrier effect, an action of ameliorating wrinkling or an action of preventing wrinkling, an action of improving skin elasticity or an action of maintaining skin elasticity, an action of ameliorating skin thickening or an action of preventing skin thickening, an action of enhancing ceramide production or an action of suppressing reduction in ceramide production, or an action of suppressing cellular growth. The cosmetics can also be cosmetics having an indication saying that the cosmetics are used for exhibiting a desired effect by, for example, an antitumor activity, an action of improving moisture retention or an action of preventing moisture retention, an action of improving the barrier effect or an action of preventing the barrier effect, an action of ameliorating wrinkling or an action of preventing wrinkling, an action of improving skin elasticity or an action of maintaining skin elasticity, an action of ameliorating skin thickening or an action of preventing skin thickening, an action of enhancing ceramide production or an action of suppressing reduction in ceramide production, or an action of suppressing cellular growth.

The content of the active ingredient of the present invention in the cosmetics of the present invention is, in usual cases, preferably 0.0001 to 20% by weight, more preferably 0.001 to 5% by weight, even more preferably 0.03 to 3% by weight, on a dry-weight basis.

If necessary, the cosmetics of the present invention can comprise, as a component other than the active ingredient of the present invention, a moisturizing agent such as 1,3-butylene glycol or pyrrolidone carboxylate; a skin softener such as liquid paraffin, petrolatum, olive oil, squalane, lanoline or synthetic ester oil; fats and oils such as coconut oil or palm oil; vitamins such as vitamin E; a surfactant such as beeswax, propylene glycol monostearate or stearic acid; an auxiliary agent for stabilizing an emulsion, such as stearyl alcohol; a solubilizer such as polyoxyethylene cetyl ether and polyoxyethylene hydrogenated castor oil; a preservative such as methylparaben; a pigment; a flavor; an antioxidant; a UV absorber; a pharmacologically active substance; a base material; a surfactant; or the like. Further, a component having a water-holding action, such as polysaccharides represented by glycerin, propylene glycol, polyethylene glycol and hyaluronic acid can also be simultaneously used.

The form of the cosmetics of the present invention is not particularly limited as long as the above-mentioned physiological action of the active ingredient can be expected, and the cosmetics of the present invention are preferably in the form of, for example, lotions, emulsions, creams, masks, a bathing agent, a face wash, bath soap, a bath detergent, or an ointment.

The cosmetics of the present invention can be properly produced according to a method known in the field of cosmetics, using the active ingredient of the present invention and, if desired, other components mentioned above, as raw materials. Cosmetics suitable for oral ingestion can also be produced according to a method known in the field of a food and beverage in the same manner as for the food, beverage etc. described later.

When the cosmetics containing the active ingredient of the present invention in the above-mentioned range are applied in a desired amount depending on the application form, for example, when the lotion is used in an amount of preferably about 0.01 to 5 g per use, more preferably about 0.1 to 2 g, onto a whole of a human face, there can be brought about an effect of retaining moisture, a barrier effect, an effect of ameliorating wrinkling or an effect of preventing wrinkling, an effect of improving skin elasticity or an effect of maintaining skin elasticity, an effect of ameliorating skin thickening or an effect of preventing skin thickening, and an effect of enhancing ceramide production or an effect of suppressing reduction in ceramide production and an effect of suppressing cellular growth, thus achieving the desired effect of the present invention, such as an effect of conferring tautness and gloss on the skin and an effect of making the skin beautiful.

The skin described herein includes every part covering the outside of a human and animal, such as face, neck, chest, back, arm, hand, leg, foot, buttock, and scalp.

(5) Food of the Present Invention
The food of the present invention comprises the ceramidase activity inhibitor of the present invention, and the desired effect of the food of the present invention is exhibited based on the above-mentioned physiological action possessed by the inhibitor (active ingredient) contained in the food. The present invention provides a food comprising the ceramidase inhibitor of the present invention, and the food is characterized in that the food comprises the ceramidase inhibitor of the present invention at a high concentration and/or in a higher purity as compared with conventional food. Ingestion of the food of the present invention can be expected to lead to an effect such as cellular growth suppression or apoptosis induction, and can produce a therapeutic or prophylactic action effectively on a disease associated with ceramide. The food can also be a health food (food for specified health use) with an indication saying that the food is used for exhibiting the desired effect by, for example, the cellular growth suppressive activity or apoptotic induction activity. The food of the present invention also includes a beverage, and for the sake of convenience, a food in the form for drinking is sometimes referred to as a beverage, and a food in the form other than that for drinking is sometimes referred to as a food.

The food of the present invention is not particularly limited, as far as the food comprises the ceramidase activity inhibitor of the present invention. The food includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao,* and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor *(shochu),* vodka; brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, bottled or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods (soups and the like); spices; and the like. These foods can be manufactured by a known method for manufacturing a food, using the ceramidase activity inhibitor of the present invention.

The content of the active ingredient of the present invention in the food of the present invention is not particularly limited and can be properly selected from the viewpoint of exhibiting its function and working effect. For example, the content of the active ingredient of the present invention in the food on a dry weight basis is preferably 0.0001 part by weight or more, more preferably 0.001 to 10 parts by weight, per 100 parts by weight of the starting material of the food itself, and the content of the active ingredient in the beverage on a dry weight basis is preferably 0.0001 part by weight or more, more preferably 0.001 to 10 parts by weight, per 100 parts by weight of the raw material of the beverage itself.

As long as the above-mentioned active ingredient or active ingredients are comprised, added and/or diluted, and depending on its application form, the content of the active ingredient(s) corresponds to a necessary amount for inhibiting the ceramidase activity, the form of the food of the present invention is not particularly limited, and includes an orally ingestible form such as a tablet, granule, a capsule, a soft capsule, liquid, and powder.

(6) Method of Inhibiting the Ceramidase Activity in the Present Invention The method of inhibiting the ceramidase activity according to the present invention is a method of inhibiting the ceramidase activity by applying the active ingredient of the present invention to a living body (for example, a mammal, a mammal-derived tissue, a mammal-derived cell, and a cell of fungi, yeast, basidiomycete or the like) or to a living body-derived sample (for example, a cell extract or a purified product thereof), and can usually be carried out by applying the ceramidase activity inhibitor, ceramide level regulator, medicament, quasi-drug, cosmetics or a food according to the present invention to the living body or living body-derived sample, depending on the application form. The application method is not particularly limited as long as the active ingredient of the present invention can be contacted with the living body or living body-derived sample, and the method includes a method such as administration of the active ingredient of the present invention to a mammal, addition of the active ingredient of the present invention to a mammal-derived cell culture.

The method of inhibiting the ceramidase activity according to the present invention is useful, for example, for treatment of cellular growth abnormality, cancer, autoimmune disease, skin immunity abnormality, infection, inflammation such as dermatitis and arthritis, wrinkle formation (mechanism of reduction in skin elasticity and skin thickening), abnormality in the barrier function of horny layer of the skin, and dry skin, for amelioration of the symptoms thereof, for study thereon, and for production and purification of ceramide.

Embodiments of the method of inhibiting the ceramidase activity in the present invention are not particularly limited as long as the active ingredient of the present invention can exhibit its inhibitory activity, and the ceramidase activity in the living body, cell, tissue and a sample derived therefrom can be inhibited.

### EXAMPLES

Hereinafter, the present invention is described in more detail by referring to the Examples, but the scope of the present invention is not limited to these examples.

### Production Example 1 Preparation of Ceramidase

As neutral/alkaline ceramidase, two kinds of ceramidase derived from *Pseudomonas aeruginosa* strain AN17 and ceramidase derived from a rat brain were used.

*Pseudomonas aeruginosa* strain AN17 is a bacterial strain separated from a skin released from a patient with atopic dermatitis and produces neutral/alkaline ceramidase (Journal of Biological Chemistry, 273: 14368-14373 (1998)). The bacterial strain was designated AN17 and has been deposited since June 26, 1996 (original deposition date) under Accession No. FERM P-15699 with International Patent Organisms Depository, National Institute of Advanced Industrial Science and Technology, located at Central 6, 1-1-1 Higashi, Tsukuba, Ibaraki Prefecture (zip code: 305-8566), Japan. A ceramidase crude enzyme solution was prepared in the following manner.

*Pseudomonas aeruginosa* strain AN17 was cultured at 30°C for 3 days in a sphingomyelin-comprising peptone yeast extract medium (0.5% peptone, 0.1% yeast extract, 0.5% NaCl, 0.01% sphingomyelin (manufactured by Sigma), 0.05% sodium taurodeoxycholate (TDC), pH 7.2) and centrifuged to give a culture supernatant. This supernatant was applied onto Q-Sepharose FF (2.5×20 cm, manufactured by Pharmacia) equilibrated previously with 50 mM Tris-HCl buffer (pH 7.5) containing 0.1% Lubrol PX (manufactured by Nacalai Tesque), then the column was washed with the same buffer at a flow rate of 2 mL/min., and the enzyme was eluted with a gradient from 0 to 1 M NaCl. The active fractions were recovered, then supplemented with bovine serum albumin (BSA) at a final concentration of 1 mg/mL and dialyzed against 50 mM Tris-HCl buffer (pH 7.5) containing 0.1% Lubrol PX to prepare a crude enzyme standard.

On the other hand, a crude enzyme solution of rat brain-derived ceramidase was prepared in the following manner. First, two pieces (3.5 g) of rat brain tissues were homogenized in 30 mL of 0.25 M sucrose solution with a Potter-Elvehjem homogenizer. The resulting homogenate was centrifuged at 700×g for 10 minutes, then the supernatant was centrifuged at 25,000xg for 10 minutes, and the resulting supernatant was further centrifuged at 100,000xg for 60 minutes to give precipitates which were then suspended in 3 mL of 25 mM Tris-HCl buffer (pH 8.0). Nine milliliters of 25 mM Tris-HCl buffer (pH 8.0) comprising 0.5% Triton X-100 was added to the resulting suspension which was then subjected to extraction by leaving the suspension on ice for 2 hours. The sample was centrifuged at 100,000xg for 60 minutes to recover a supernatant as a crude enzyme standard.

Example 1 Preparation of *Wakame* Seaweed (*Undaria pinnatifida*) Sporophyll Extract
One hundred milliliters of ethanol was added to 10 g of dry *wakame* (*Undaria pinnatifida*) sporophyll chips, and the mixture was stirred gently several times and then left for 1 day as it was. The mixture was filtered to give a *wakame* (*Undaria pinnatifida*) sporophyll ethanol extract. The dry weight per 1 mL of extract was 4 mg.

Example 2 Preparation of a Kelp (*Laminaria japonica* Areschoug) Extract and a Gagome (*Kjellmaniella crassifolia* Miyabe) Extract
One hundred milliliters of ethanol was added to 10 g of dry kelp (*Laminaria japonica* Areschoug) chips, and the mixture was stirred gently several times and then left for 1 day as it was. The mixture was filtered to give a kelp (*Laminaria japonica* Areschoug) ethano 1 extract. The dry weight per 1 mL of extract was 2.7 mg.

An extract of gagome (*Kjellmaniella crassifolia* Miyabe) was also obtained in a same manner. The dry weight per 1 mL of extract was 1.8 mg.

Example 3 Preparation of Various Plant Extracts
Ginkgo (*Ginkgo biloba*) leaves, white muskmelon *(Cucumis melo L.)* fruits, wax gourd *(Benincasa cerifera* Savi) fruits, cucumber *(Cucumis sativus* L.) fruits, bitter cucumber (*Momordica charantia* L.) fruits, or mugwort *(Artemisia vulgaris* L. var. *indica* Maxim) leaves were formed into a juice by a homogenizer and then lyophilized, followed by adding 10 mL of ethanol per 1 g of the lyophilized product and leaving it overnight for extraction. The sample was filtered to give an ethanol extract. The dry weight per 1 mL of the ginkgo *(Ginkgo biloba*) leave extract was 15 mg, the dry weight per 1 mL of the white muskmelon *(Cucumis melo L.)* extract was 120 mg, the dry weight per 1 mL of the wax gourd (*Benincasa cerifera* Savi) extract was 53 mg, the dry weight per 1 mL of the cucumber (*Cucumis sativus* L.) extract was 45 mg, the dry weight per 1 mL of the bitter cucumber (*Momordica charantia* L.) extract was 42 mg, and the dry weight per 1 mL of the mugwort *(Artemisia vulgaris* L. var. *indica* Maxim) extract was 43 mg.

For plant essential oil such as orange oil or grapefruit oil, those commercially available from Yamamoto Koryo Co., Ltd. were used.

Reference Example 1 Determination of Ceramidase Inhibitory Activity
The inhibitory activity on neutral/alkaline *Pseudomonas aeruginosa* Ceramidase was determined in the following manner. First, 10 µL of a diluted enzyme solution prepared by diluting the *Pseudomonas aeruginosa* Ceramidase at a proper enzyme concentration with a diluent buffer (100 mM Tris-HCl buffer, pH 8.5, containing 5 mM calcium chloride, 0.45% bovine serum albumin) was mixed with 5 µL of inhibitor and kept at 37°C for 10 minutes. Ten microliters of a substrate solution (Tris-HCl buffer, pH 8.5, containing 0.05 mM or 1.5 mM NBD-C12-Ceramide (manufactured by Matreya Inc.), 5 mM CaCl₂, 0.5% Triton X-100) was added thereto and reacted at 37°C for 30 minutes. The reaction was terminated by adding 75 µL of methanol, and 25 µL of aliquot of the reaction solution was analyzed by HPLC.

The inhibitory activity on the neutral/alkaline RatBrain Ceramidase was determined in the following manner. First, 10 µL of a diluted enzyme solution prepared by diluting the RatBrain Ceramidase at a suitable enzyme concentration with a diluent buffer (100 mM glycine-NaOH buffer, pH 9.5, containing 12.5 mM magnesium chloride) was mixed with 5 µL of inhibitor and kept at 37°C for 10 minutes. Ten microliters of a substrate solution (100 mM glycine-NaOH buffer, pH 9.5, containing 0.05 mM or 1.5 mM NBD-C12-Ceramide, 1.25% taurodeoxycholate) was added thereto and reacted at 37°C for 2 hours. The reaction was terminated by adding 75 microliters of methanol, and 50 microliters of aliquot of the reaction solution was analyzed by HPLC.

HPLC analysis was carried out in the following manner. The column used was Cosmosil 5C18-AR-II, 4.6x50 mm (manufactured by Nacalai Tesque), and the sample was eluted by isocratic elution with MeOH/1% TFA = 90 : 10 (v/v) as an eluent. The flow rate was 1 mL/min. Detection was carried out with a fluorescence detector at an excitation light of 465 nm and a fluorescent light of 535 nm.

In the case of either enzyme, a combination of the enzyme + inhibitor + substrate was reacted as a sample; a combination of the enzyme + substrate, as a control; and a combination of the inhibitor + substrate, as a blank. The ratio of inhibition was calculated from the activity of the sample, assuming that the control activity was 100%.

When the method in Reference Example 1 is merely referred to herein, the method using neutral/alkaline RatBrain Ceramidase is intended.

Example 4 Inhibitory Effect of Seaweed Extracts, Plant Extracts or the like on Ceramidase
Using the method described in Reference Example 1, the ceramidase-inhibiting activities of the various seaweed, plant extracts or the like obtained in Examples 1 to 3 were determined using NBD-C12-Ceramide at a final concentration of 0.6 mM as a substrate. The results are shown in Table 1. Dilution of the seaweed, plant extracts and orange oil (limonene) was carried out with dimethyl sulfoxide.

**[Table 1]**

| | | Ceramidase Inhibition(%) | |
|---|---|---|---|
| Plant Extract etc. | Dilution Ratio | *P. aeruginosa* Ceramidase | RatBrain ceramidase |
| Ginkgo | 2 | 77.9 | 63.1 |
| (*Ginkgo biloba*) | 5 | 28.1 | 53.1 |
| | 10 | 8.5 | 53.9 |
| Wax gourd | 2 | 76.3 | 74.9 |
| (*Benincasa cerifera* | *5* | 58.5 | 82.2 |
| Savi) | 10 | 44.9 | 76.5 |
| Bitter cucumber | 2 | 76.6 | 60.5 |
| (*Momordica charantia* | 5 | 70.3 | 80.0 |
| L.) | 10 | 61.8 | 75.1 |
| Oriental pickling melon | 2 | 69.1 | 69.3 |
| (*Cucumis melo* L. var. | 5 | 69.0 | 85.0 |
| *conomon* Makino) | 10 | 49.9 | 83.0 |
| Cucumber | 2 | 34.3 | 65.9 |
| *(Cucumis sativus* L.) | 5 | 31.7 | 57.4 |
| | 10 | 15.6 | 49.4 |
| Mugwort | 2 | 29.4 | 51.3 |
| (*Artemisia vulgaris* L. | 5 | 13.4 | 43.1 |
| var *indica* Maxim.) | 10 | 5.4 | 42.3 |
| Kelp | 10 | 83.8 | 83.3 |
| (*Laminaria japonica* | 20 | 69.9 | 82.6 |
| Areschoug) | 50 | 41.4 | 78.4 |
| Gagome | 10 | 60.5 | 78.7 |
| (*Kjellmaniella* | 20 | 44.2 | 73.8 |
| *crassifolia* Miyabe) | 50 | 35.9 | 67.9 |
| Orange oil (*Citrus sinensis, Citrus* | 10 | 16.9 | 53.9 |
| *aurantium* or *Citrus reticulate)* | 20 | 18.1 | 47.7 |

Example 5 Inhibitory Effect of *Wakame* (*Undaria pinnatifida*) Sporophyll Extract on Ceramidase
One gram of dry *wakame* (*Undaria pinnatifida*) sporophyll chips were extracted overnight with 5 mL of 100% ethanol, 50% ethanol and 25% ethanol solution respectively and then filtered to give *wakame* (*Undaria pinnatifida*) sporophyll extracts. The dry weights of the resulting extracts per 1 mL were 8 mg, 72 mg and 74 mg, respectively. Using the method described in Reference Example 1, the inhibitory activity of each *wakame* (*Undaria pinnatifida*) sporophyll extract was determined using NBD-C12-Ceramide at a final concentration of 0.02 mM as a substrate. The ratios of inhibition of ceramidase by 10-fold dilutions of the extracts were 71%, 43% and 11%, respectively.

Example 6 Effect of the Seaweed and Plant Extracts on Cultured Human Leukemia Cells (TLC)
Human promyelocytic leukemia cell line, HL60 was cultured in RPMI-1640 medium containing 10% bovine fetal serum and 1% penicillin/streptomycin solution (Lot No. 20K1346 manufactured by GIBCO BRL) at 37°C in a 5% CO₂ environment. The cultured cells were recovered by centrifugation at 1500 rpm, the cell pellet was washed with UltraDOMA-PF medium (manufactured by BioWhittaker), and the cells were counted and adjusted finally to a density of 2×10e⁷ cells/1.8 mL, pipetted to a 6-well plate in a volume of 1.8 mL/well, and cultured under the same environment for 2 hours. Two milliliters each of the various seaweed and plant extracts obtained in Examples 1 to 3 were concentrated with a centrifugal concentrator to remove the solvent, and each extract was dissolved in 40 µL of dimethyl sulfoxide (DMSO) and 1.8 mL of UltraDOMA medium was added thereto. Zero point two milliliter of this extract dilution was added to 1.8 mL of the cultured cells, which were then cultured at 37°C in a 5% CO₂ environment for up to 3 hours. Only the same diluent component as used in preparing the extraction dilution was added to the control group. A sample for counting viable cells and a sample for determining sphingolipid were prepared by sampling for determination in the following manner.

The cultured cell suspension was recovered with a micropipette into a 10-mL glass tube equipped with a screw cap, and 40 µL of the cell culture was used as a sample for counting viable cells. One hundred and forty microliters of UltraDOMA medium and 20 µL of trypan blue were added thereto and the viable cells were counted with a hemocytometer. The remainder of the cell suspension was used to prepare a sample for determining sphingolipid. The cultured cell suspension was centrifuged at 1,500 rpm for 5 minutes, and the cell pellet was washed 3 times with 1 mL of PBS. One milliliter of Hanks buffer (HBSS, manufactured by Life Technologies, Inc.) containing 0.1% bovine serum albumin (BSA) was added to the washed cell pellet and stored at -30°C until analysis as a sample for lipid analysis.

Extraction of lipid from the analysis sample was carried out in the following manner.
Three milliliters of chloroform/methanol = 2 : 1 (v/v) was added to 1 mL of analysis sample and stirred well for 20 minutes in a shaker. The sample was centrifuged at 3,000 rpm for 2 minutes, and the lower layer was transferred to another glass tube and then dried by completely distilling the solvent off with a centrifugal concentrator. The resulting sample was stored at -30°C until analysis as a sample for thin-layer chromatography (TLC) analysis. TLC analysis was carried out under the following conditions.

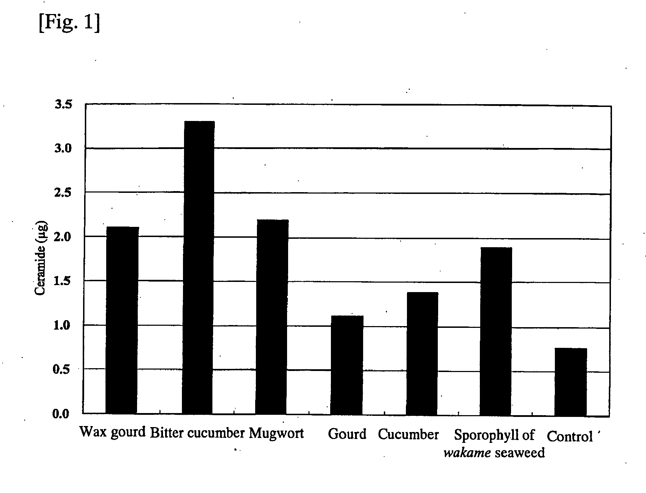

The TLC analysis sample was dissolved by adding 20 µL of chloroform/methanol = 1 : 1 (v/v), and the whole solution was applied onto a TLC plate (HPTLC plate silica gel 60F254, manufactured by Merck). As ceramide standards, non-hydroxy fatty acid ceramide or non-hydroxyceramide (ceramide type III, manufactured by Sigma-Aldrich) and hydroxy fatty acid ceramide or hydroxyceramide (ceramide type IV, manufactured by Sigma-Aldrich) were used. The sample was developed twice with chloroform/methanol/acetic acid = 190 : 9 : 1 (v/v) as a developing solvent, and then the TLC plate was sprayed with a phosphoric acid/copper reagent (8% phosphoric acid solution containing 10% CuSO₃) and heated at 160°C for 10 minutes, and the resulting spots were quantified by a densitometer. The results are shown in Fig. 1. The groups to which the extract of wax gourd (*Benincasa cerifera* Savi), bitter cucumber (*Momordica charantia* L.), mugwort (*Artemisia vulgaris* L. var *indica* Maxim.), gourd, cucumber (*Cucumis sativus* L.) or *wakame* (*Undaria pinnatifida*) sporophyll had been added showed an increase in intracellular ceramide level as compared with the control.

Example 7 Effect of *Wakame* (*Undaria pinnatifida*)Sporophyl1 Extract on Cultured Human Leukemic Cells (LCMS)
Human promyelocytic leukemia cell line HL60 was cultured in an RPMI-1640 medium containing 10% bovine fetal serum and 1% penicillin/streptomycin solution (Lot No. 20K1346 manufactured by GIBCO BRL) at 37°C in a 5% CO₂ environment. The cultured cells were recovered by centrifugation at 1500 rpm, the cell pellet was washed with UltraDOMA-PF medium (manufactured by Bio Whittaker), and the cells were counted and adjusted finally to a density of 1×10e⁷ cells/1.8 mL, pipetted to a 6-well plate in a volume of 1.8 mL/well and further cultured under the same environment for 2 hours. The *wakame* (*Undaria pinnatifida*) sporophyll extract used was prepared by removing the solvent from 2 mL of extract with a centrifugal concentrator, then adding 40 µL of dimethyl sulfoxide (DMSO) to dissolve it, and adding 1.8 mL of UltraDOMA medium thereto at a DMSO concentration of 2%. Zero point two milliliter of this plant extract dilution was added to 1.8 mL of the above-mentioned cultured cells which were then cultured at 37°C under a 5% CO₂ environment for up to 24 hours. With respect to the control group, only the same diluent component as used in preparing the extract dilution was added to the cultured cells. The cultured cells were sampled for determination at 0 hour, 0.5 hour, 1 hour and 3 hours later respectively to prepare a sample for counting viable cells and a sample for determining sphingolipid.

The cultured cell suspension was recovered with a micropipette into a 10-mL glass tube with a screw cap, and 40 µL of aliquot was sampled for counting viable cells. One hundred and forty microliters of UltraDOMA medium and 20 µL of trypan blue were added thereto and the viable cells were counted with a hemocytometer. The remaining cells were used to quantify sphingolipid according to a method of Mano et al. (Analytical Biochemistry, 244: 291-300, 1997). The cultured cell suspension was centrifuged at 1,500 rpm for 5 minutes, and the cell pellet was washed 3 times with 1 mL of PBS. One milliliter of Hanks buffer containing 0.1% bovine serum albumin (BSA) was added to the washed cell pellet and stored at -30°C until analysis as a lipid analysis sample.

A sphingolipid standard for quantification used in LCMS was prepared in the following manner.
All sphingolipids were dissolved in methanol. Three hundred microliters of 1 mg/mL C18-sphingomyelin (C18-SM, manufactured by Matorea), 100 µL of 10 µg/mL C18-ceramide (C18-Cer, manufactured by Matorea), 100 µL of 10 µg/mL sphingosine (Sph, manufactured by Sigma), 100 µL of 3 µg/mL sphingosyl phosphoryl choline (SPC, manufactured by Sigma), 100 µL of 1 µg/mL dimethyl sphingosine (DMS, manufactured by Avanti Lipid), 100 µL of 1 µg/mL psychosine (Psy, manufactured by Sigma), and 200 µL of methanol were mixed to give 1 mL of mixture for use as a standard stock solution. This solution was further diluted with methanol to prepare a 10-fold dilution and 100-fold dilution, respectively. One hundred microliters of each dilution was added to 900 µL of HBSS containing 0.1% BSA, sufficiently stirred and stored as a sphingolipid standard mixture at -30°C until use.

Extraction of lipid from the analysis sample and the standard was carried out in the following manner.
Three milliliters of chloroform/methanol = 2 : 1 (v/v) was added to 1 milliliter of analysis sample, and 100 µL of 1 µg/mL C2-ceramide (C2-Cer, manufactured by Matorea) in methanol was added as an internal standard and sufficiently stirred for 10 minutes with a Vortex mixer. The sample was centrifuged at 3,000 rpm for 2 minutes, and the lower layer was transferred to another glass tube, and the solvent was completely distilled off in a centrifugal concentrator to dry the sample. This sample was stored at -30°C until analysis as an LCMS analysis sample.

LCMS was carried out under the following conditions.
The HPLC column used was a semi-micro short column, YMC-Pack Pro C18 (2.0x35 mm, 3 µm, manufactured by YMC). Eluent A was 5 mM ammonium formate/methanol/tetrahydrofuran = 5 : 2 : 3 (v/v/v) (comprising formic acid at a final concentration of 0.01%), and eluent B was 5 mM ammonium formate/methanol/tetrahydrofuran = 1 : 2: 7 (v/v/v) (comprising formic acid at a final concentration of 0.01%). The eluate from HPLC was introduced via a splitter into an ion-spray quadrupole mass spectrometer (API 300, manufactured by Applied Biosystems). With an orifice voltage of 70 eV, an ion-spray voltage of 5000 V, in a positive mode, the determination was carried out using an argon gas as collision gas in a multiple reaction monitoring mode. Monitoring ions are shown in Table 2.

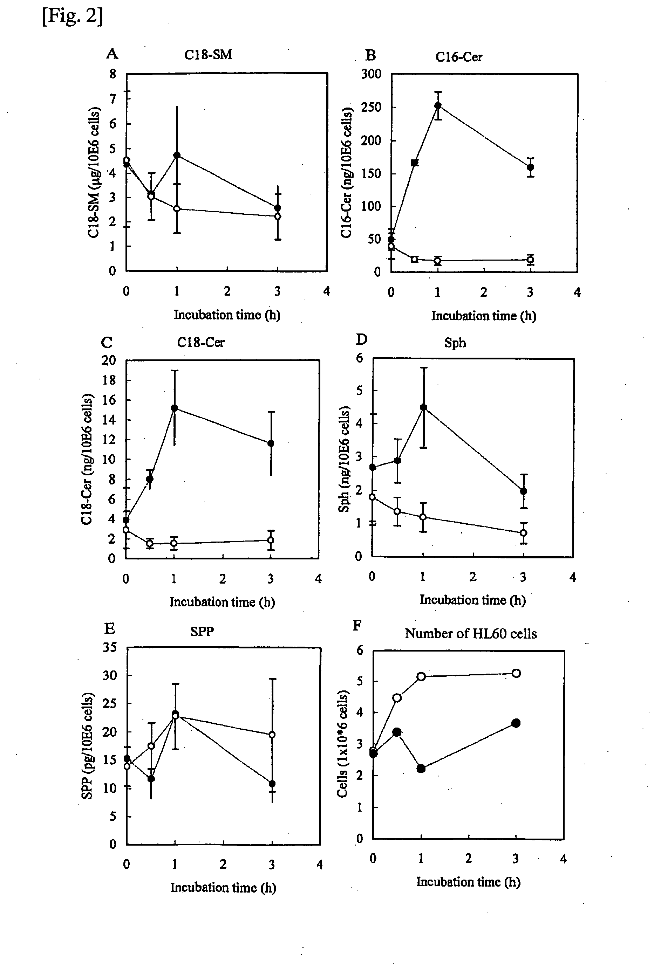

The results are shown in Fig. 2. In the figure, the group to which the *wakame* (*Undaria pinnatifida*) sporophyll extract was added is shown in the filled circle, and the control group is shown in the open circle. Graph A shows C18-sphingomyelin (C18-SM); B, C-16 ceramide (C16-Cer); C, C18-ceramide (C18-Cer); D, sphingosine (Sph); E, sphingosine-1-phosphate (SPP); and F, the number of HL60 cells. In the group to which the *wakame* (*Undaria pinnatifida*) sporophyll extract was added, the intracellular C16-Cer and C18-Cer were increased to be about 15 times and about 10 times respectively as high as those of the control group. On the other hand, Sph, that is, a degradation product of ceramide, was increased to be about 4 times as high as that of the control group. Sphingosine-1-phosphate, that is, a metabolite of sphingosine, tended to be slightly lower in the group to which the *wakame* (*Undaria pinnatifida*) sporophyll extract had been added than the control group. The C18-SM level did not change. Growth of HL60 cells was significantly suppressed in the group to which the *wakame* (*Undaria pinnatifida*) sporophyll extract had been added, as compared with the control group. From the cells, DNA was extracted by ApopLadder Ex (manufactured by TAKARA BIO INC.), then subjected to electrophoresis on 1% agarose gel and stained with ethidium bromide, and as a result, fragmented DNA ladders were detected in the group to which the *wakame* (*Undaria pinnatifida*) sporophyll extract had been added, thus revealing that the cells had undergone apoptosis. From these results, it was revealed that by the ceramidase-inhibiting effect of the *wakame* (*Undaria pinnatifida*) sporophyll extract, the intracellular ceramide levels were increased, cellular growth was inhibited, and the cells underwent apoptosis.

**[Table 2]**

| LCMS Multiple Reaction Monitoring (MRM) | | |
|---|---|---|
| Compound | Monitoring Ions | |
| | Precursor Ion | Product Ion |
| C20-SM | 760 | 184 |
| C18-SM | 732 | 184 |
| C16-SM | 704 | 184 |
| C20-Cer | 594 | 264 |
| C18-Cer | 566 | 264 |
| C16-Cer | 538 | 264 |
| C17:1-Cer | 550 | 264 |
| C2-Cer | 342 | 264 |
| SPC | 465 | 184 |
| Psy | 462 | 282 |
| DMS | 328 | 310 |
| Sph | 300 | 282 |
| SPP | 380 | 264 |

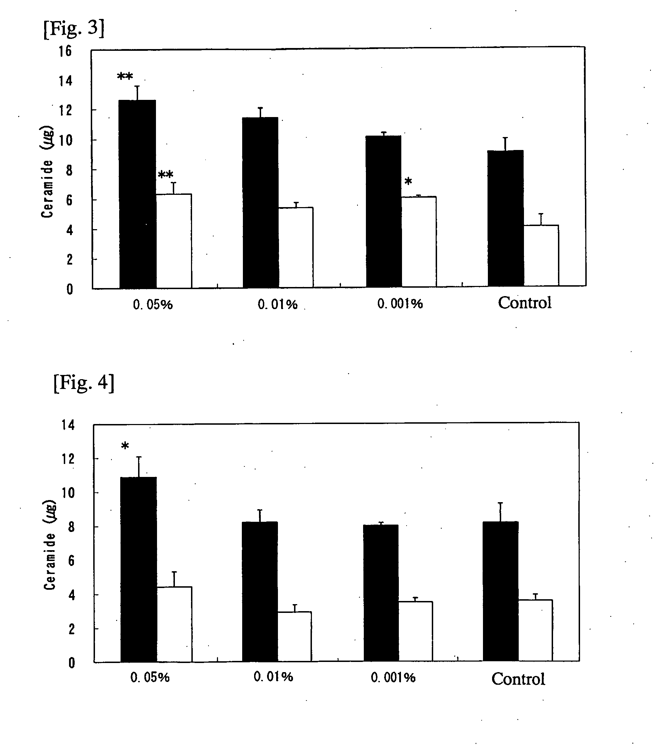

Example 8 Effect of the Ceramidase Inhibitor in Artificial Cultured Skin
Using a commercial normal human skin 3-dimensional cultured model (EPI-100, manufactured by KURABO INDUSTRIES LTD.), the effect of the ceramidase inhibitor on skin cells was determined. First, a mini cup of EPI-100 was put to each well containing 2 mL of maintenance medium for EPI-100 (gentamicin-free medium) in a 6-well culture plate, and was cultured at 37°C in a 5% CO₂ environment for 72 hours. After the medium was exchanged with 4 mL of fresh medium, 50 µL of dilution of the *wakame* (*Undaria pinnatifida*) sporophyll ethanol extract obtained in Example 1 was added to the mini cup. The dilution of the *wakame* (*Undaria pinnatifida*) sporophyll ethanol extract used was obtained by once removing the solvent from the *wakame* (*Undaria pinnatifida*) sporophyll ethanol extract in an evaporator and then redissolving the extract in a vehicle (80% propylene glycol containing 0.5% polyoxyethylene hydrogenated castor oil) such that the concentration of the extract became 0.05% to 0.001% (w/v). The sample to which the only vehicle was added was used as a control. The cells were cultured at 37°C in a 5% CO₂ environment for 24 hours, then the medium was exchanged with fresh one, the ethanol extract was added to the mini cup, and the cells were cultured for 24 hours. After exchange of the medium and addition of the ethanol extract were carried out again, the cells were cultured for 24 hours, and the mini cup was removed to determine ceramide in the skin cells. First, the cup was washed with physiological saline, and the cultured skin was removed with tweezers, transferred to a glass test tube equipped with a screw cap, and lyophilized. Two milliliters of chloroform/methanol (2 : 1) was added to the sample, and extraction was carried out under occasional stirring at room temperature for 2 hours, to extract a ceramide fraction. The sample was centrifuged at 2,000 rpm for 5 minutes, and the supernatant was transferred to another glass tube (supernatant 1). Precipitates were dried with a centrifugal concentrator and then subjected to extraction again with 2 mL of chloroform/methanol (2 : 1) and centrifuged to give a supernatant (supernatant 2). The supernatants 1 and 2 were combined, the solvent was then removed, and the combined sample was stored at -30°C until analysis as a sample for thin-layer chromatography (TLC) analysis. TLC analysis was carried out in the same manner as in Example 6. The results are shown in Fig. 3. In the figure, the black bar shows non-hydroxyceramide, and the white bar shows hydroxyceramide. Both non-hydroxyceramide and hydroxyceramide in the artificial cultured skin were increased significantly depending on the concentration of the added extract in the group to which the *wakame* (*Undaria pinnatifida*) sporophyll ethanol extract had been added, as compared with the control group. In Fig. 3, * indicates p < 0.05 and * * indicates p < 0.005 in t-test relative to the control.

Example 9 Effect of the Ceramidase Inhibitor on an Artificial Cultured Skin Model Infected with *Pseudomonas aeruginosa*
It has been noted that in the surface of the skin of a patient with atopic dermatitis, ceramide connecting cells in a horny layer is reduced, and this reduction causes deficient in barrier function of the skin, that is, causes invasion of foreign antigen into the living body and excessive water loss out of the body, which in its turn may be associated with onset and worsening of this disease. Recently, Ito et al. in Kyushu University, Japan, have reported that ceramidase-producing microorganisms are detected with statically significant frequency in the skin of a patient with atopic dermatitis, and a majority of these microorganisms are *Pseudomonas aeruginosa (*J. Biol. Chem., 273: 14368-14373 (1998) and Clinc. Diagnost. Lab. Immun., 6: 101-104 (1999)). On the other hand, it has been well known that infection with *Staphylococcus aureus* is found with high frequency in the skin of a patient with atopic dermatitis. Ito et al. have also reported that the ceramidase-producing microorganisms produce a protease which lyses *Staphylococcus aureus,* and from the lysed *Staphylococcus aureus,* anionic glycerophospholipids which activate ceramidase, such as cardiolipin and phosphatidyl glycerol, are eluted *(*Biochem. J., 362: 619-626 (2002)). These results suggest a possibility that upon infection of the skin of a patient with atopic dermatitis with the ceramidase-secreting microorganism *Pseudomonas aeruginosa, Staphylococcus aureus* is lysed, and the glycerophospholipids eluted therefrom activate ceramidase to reduce the ceramide level in the horny layer of the skin.

Accordingly, the effect of the ceramidase inhibitor was evaluated by adding the ceramidase inhibitor to a system in which a normal human skin 3-dimensional cultured model (EPI-100) had been infected with *Pseudomonas aeruginosa.*

First, *Pseudomonas aeruginosa* strain AN17 was cultured in L-broth (manufactured by TAKARA BIO INC.) at 30°C, and when the value at A660 nm reached 0.6, culture was finished and the bacteria were recovered. The bacteria were washed with PBS and then suspended at 4×10⁷ cfu/mL with PBS. Separately, *Staphylococcus aureus* was also cultured in L-broth at 30°C, and when the value at A660 nm reached 0.6, culture was finished and the bacteria were recovered. The bacteria were washed with PBS, suspended at 20×10⁷ cfu/mL with PBS and treated at 100°C for 5 minutes to prepare dead bacteria. This dead *Staphylococcus aureus* suspension was diluted 5-fold with PBS, and the resulting dilution and the *Pseudomonas aeruginosa* suspension in PBS were mixed in equal amounts just before use. A mini cup of EPI-100 was put to each well containing 0.9 mL of maintenance medium for EPI-100 (gentamicin-free medium) in a 6-well culture plate, and pre-cultured at 37°C in a 5% CO₂ environment for 2 hours. After the medium was exchanged with 2 mL of fresh maintenance medium, 50 µL of bacterial suspension was added to the mini cup and the bacteria were cultured at 37°C in a 5% CO₂ environment for 72 hours. After the medium was exchanged with 4 mL of fresh maintenance medium, 50 µL of the *wakame* (*Undaria pinnatifida*) sporophyll ethanol extract dilution used in Example 8 was added thereto. The sample to which only the vehicle was added was used as a control. The skin cells were cultured at 37°C in a 5% CO₂ environment for 24 hours, then the medium was exchanged with fresh one, the ethanol extract was added to the mini cup, and the skin cells were cultured for 24 hours. After exchange of the medium and addition of the ethanol extract were carried out again, the cells were cultured for 24 hours, and the mini cup was then removed to determine ceramide in the skin cells. First, the cup was washed with physiological saline, and the cultured skin was then removed with tweezers, transferred to a glass test tube equipped with a screw cap, and lyophilized. Two milliliters of chloroform/methanol (2 : 1) was added to the sample, which was then extracted under occasional stirring at room temperature for 2 hours to give a ceramide fraction. The sample was centrifuged at 2,000 rpm for 5 minutes, and the supernatant was transferred to another glass tube (supernatant 1). Precipitates were dried with a centrifugal concentrator and then subjected to extraction again with 2 mL of chloroform/methanol (2 : 1) and centrifuged to give a supernatant (supernatant 2). The supernatants 1 and 2 were combined, the solvent was then removed, and the combined sample was stored at -30°C as a sample for thin-layer chromatography (TLC) analysis until analysis. TLC analysis was carried out in the same manner as in Example 6. The results are shown in Fig. 4. In the figure, the black bar shows non-hydroxyceramide, and the white bar shows hydroxyceramide. Non-hydroxyceramide in the artificial cultured skin was increased significantly in the group to which 0.05% *wakame* (*Undaria pinnatifida*) sporophyll ethanol extract had been added, as compared with the control group. Hydroxyceramide also tended to be increased. The ceramide level in the group to which 0.05% *wakame* (*Undaria pinnatifida*) sporophyll ethanol extract had been added was increased than the ceramide level in the control (see Fig. 3) in Example 8 to which *Pseudomonas aeruginosa* had not been added, indicating that the *wakame* (*Undaria pinnatifida*) sporophyll extract recovered the reduction in ceramide by ceramidase in *Pseudomonas aeruginosa,* and also inhibits ceramidase in the skin horny layer, thereby further increasing the ceramide level in the horny layer. In Fig. 4, * indicates p < 0.05 in t-test relative to the control.

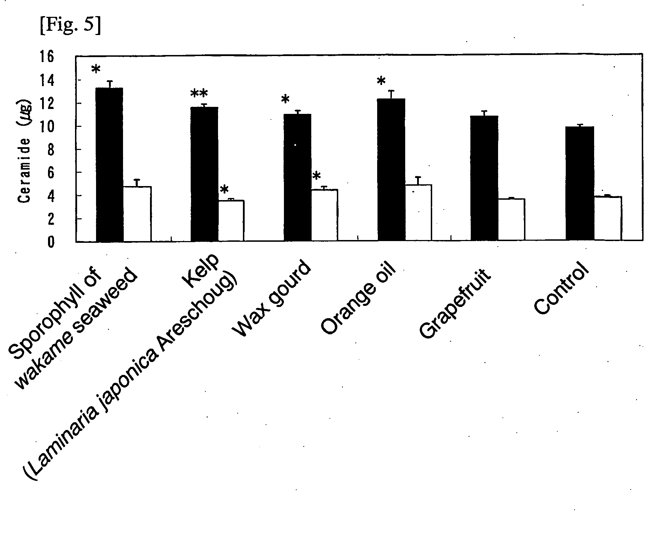

Example 10 Effect of the Ceramidase Inhibitor on an Artificial Cultured Skin Model Infected with *Pseudomonas aeruginosa*
The effects of various plant-derived extracts were evaluated in the same manner as in Example 9. The *wakame* (*Undaria pinnatifida*) sporophyll ethanol extract used was prepared by removing the solvent from the extract obtained in Example 1 and then redissolving the resulting sample in a vehicle (80% propylene glycol containing 0.5% polyoxyethylene hydrogenated castor oil) such that the concentration of the extract became 0.05% (w/v). The kelp (*Laminaria japonica* Areschoug) extract used was prepared by removing the solvent from the extract obtained in Example 2 and then redissolving the resulting sample in the vehicle such that the concentration of the extract became 0.05% (w/v). The wax gourd (*Benincasa cerifera* Savi) extract used was prepared by removing the solvent from the extract obtained in Example 3 and then diluting the resulting sample in the vehicle such that the concentration of the extract became 0.5% (w/v). Orange oil (manufactured by Yamamoto Koryo) was used after 1000-fold dilution with the vehicle. A grapefruit extract stock solution (extracted with 80% propylene glycol, manufactured by Yamamoto Koryo) was used as it was. The sample to which the vehicle only was added was used as a control. Other methods were the same as in Example 9. The results are shown in Fig. 5. In the figure, the black bar shows non-hydroxyceramide, and the white bar shows hydroxyceramide. Non-hydroxyceramide levels in the artificial cultured skin were significantly increased and hydroxyceramide levels tended to be increased in the group to which the *wakame* (*Undaria pinnatifida*) sporophyll extract was added, the group to which the kelp (*Laminaria japonica* Areschoug) extract was added, the group to which the wax gourd (*Benincasa cerifera* Savi) extract was added, and the group to which the orange oil was added, as compared with the control group. Ceramide levels tended to be increased in the group to which the grapefruit extract was added. In Fig. 5, * indicates p < 0.05 and * * indicates p < 0.005 in t-test relative to the control.

Example 11 Purification of the Ceramidase Inhibitor from a *Wakame* (*Undaria pinnatifida*) Sporophyll Extract
(1) Ten kilograms of dry *wakame* (*Undaria pinnatifida*) sporophyll chips and 20 L of ethanol were added to a stainless steel container, then the mixture was stirred gently several times and left for 1 day as it was. The mixture was filtered and washed with ethanol, to give 34.4 L of *wakame* (*Undaria pinnatifida*) sporophyll ethanol extract.

(2) A glass column (2.5 L) was charged with DIAION HP20 manufactured by Mitsubishi Chemical Co., Ltd. The column was washed with EtOH and then equilibrated with 50% EtOH. To 34.4 L of the *wakame* (*Undaria pinnatifida*) sporophyll ethanol extract was added the same volume of water, and the resulting 50% EtOH solution (68.8 L) was applied to the column. After the whole volume was applied, the column was washed with 12.5 L of 50% EtOH and elution was carried out with 25 L of EtOH. When the inhibitory activities of the passing fraction, washing fraction, and eluted fraction were determined respectively, the activity was recognized in the fraction eluted with EtOH, and thus this fraction was concentrated to remove EtOH. The yield was 136 g.

(3) A glass column (3.5×37 cm, 350 mL) was charged with Silica Gel 60 (manufactured by Merck), and the column was equilibrated with 1 L of n-hexane. n-Hexane was added to the active fraction in the above (2), to give 300 mL of a solution, which was then applied onto the column. After the column was washed with 500 mL of n-hexane and 1L of chloroform, elution was carried out with 1 L of C/M = 9:1 and then with 1 L of C/M = 8 : 2. About 80% of the inhibitory activity was eluted in the fraction with C/M=9:1.

(4) After Ultrapack Silica Gel 60 (37x300 mm, 322 mL, manufactured by Yamazen) was washed with 1 L of n-hexane at a flow rate of 10 mL/min and then equilibrated with 1 L of chloroform, the active fraction in the above (3) (50 mL of solution prepared by evaporating the above fraction to dryness and then dissolving the residues in chloroform) was applied onto the silica gel column. After the column was washed with 1.5 L of chloroform, elution was carried out with a gradient of from chloroform to ethyl acetate (0 to 100% ethyl acetate/150 min). Finally, the column was washed with 500 mL of ethyl acetate. The inhibitory activity was eluted in 2 different fractions.
The respective fractions were combined and the solvent was removed.
Fraction I: yield 1925 mg
Fraction II: yield 440 mg

(5) Fraction I obtained by the above silica gel chromatography was further purified by reverse phase HPLC. First, Cosmosil 5C18AR (10 mm×250 mm, manufactured by Nacalai Tesque) was used. Fraction I was dissolved in 10 mL of 50% isopropanol and divided into 5 portions, and the portions were subjected respectively to chromatography. As the eluting solvent, solvent A (50% isopropanol) and solvent B (100% isopropanol) were used, and the proportion of solvent B was 0% in 0 to 20 minutes, then changed from 0 to 100% in 20 to 60 minutes and kept at 100% in 60 to 80 minutes. The flow rate was 2 mL/min and the eluate was fractionated at 1-minute intervals. The ceramidase-inhibiting activity of each fraction was determined by the method of Reference Example 1 (NBD-C12-Ceramide was used at a final concentration of 0.02 mM; this applies hereinafter). The inhibitory activity was detected in fractions 51 to 70. The active fractions were combined and the solvent was removed.
The yield was 450 mg.

(6) Three tenth of the fraction obtained in item (5) above was further purified with YMC AM-322 (10 mm×150 mm, manufactured by YMC). The sample was dissolved in isopropanol. As the eluting solvent, solvent A (50% isopropanol) and solvent B (100% isopropanol) were used, and the proportion of solvent B was 0% in 0 to 10 minutes, then changed from 0 to 100% in 10 to 50 minutes and kept at 100% in 50 to 55 minutes. The flow rate was 2 mL/min, and 20 minutes after the sample was injected, the eluate was fractionated at 1-minute intervals. The ceramidase-inhibiting activity of each fraction was determined by the method of Reference Example 1. The inhibitory activity was detected in fractions 15 to 35. The active fractions were combined and the solvent was removed. The yield was 32 mg.

(7) The fraction obtained in item (6) above was further purified with XTerra RP18 (4.6 mm×150 mm, manufactured by Waters). The sample was dissolved in isopropanol and divided into 2 portions, and the portions were subjected respectively to chromatography. As the eluting solvent, solvent A (50% isopropanol) and solvent B (100% isopropanol) were used, and the proportion of solvent B was 0% in 0 to 10 minutes, then changed from 0 to 100% in 10 to 50 minutes and kept at 100% in 50 to 55 minutes. The flow rate was 1 mL/min, and 20 minutes after the sample was injected, the eluate was fractionated at 30-second intervals. The ceramidase-inhibiting activity of each fraction was determined by the method of Reference Example 1. The inhibitory activity was detected in 2 fractions, that is, fraction I (fractions 13 to 20) and fraction II (fractions 21 to 25). The active fractions were combined and the solvent was removed. The yield was 10 mg in fraction I and 6 mg in fraction II.

(8) The fraction I obtained in item (7) above was further purified by Symmetry Shield RP18 (4.6 mm×150 mm, manufactured by Waters). The sample was dissolved in chloroform/ethanol (1 : 1). As the eluting solvent, solvent A (50% isopropanol) and solvent B (100% isopropanol) were used, and the proportion of solvent B was changed from 30 to 70% in 0 to 30 minutes and kept at 100% in 30 to 35 minutes. The flow rate was 1 mL/min and detection was carried out at 220 nm, and 10 minutes after the sample was injected, the eluate was fractionated at 15-second intervals. The ceramidase-inhibiting activity of each fraction was determined by the method of Reference Example 1. The inhibitory activity was detected in 3 fractions, that is, fraction I-I (fractions 26 to 29), fraction I-II (fractions 30 to 35) and fraction I-III (fractions 36 to 42). The active fractions were combined and the solvent was removed. The fraction I-III (yield 3.3 mg) was analyzed for its structure by NMR analysis and mass spectrometry analysis.

(9) The fraction I-III obtained in item (8) above was subjected to mass spectrometry analysis by using an ion-spray mass spectrometer (API-III, manufactured by Applied Biosystems). As a solvent, 80% acetonitrile containing 0.1 % formic acid was used and analysis was carried out in a positive mode. As a result, m/z 565 (M+H)+ signal was detected. The mass spectrum is shown in Fig. 6. By MS/MS analysis with this ion as a parent ion, signals of m/z 547 and m/z 338 were detected as daughter ions. Fig. 7 shows the mass spectrum in MS/MS analysis. In Figs. 6 and 7, the m/z value is shown on the axis of abscissas and the relative intensity of signal on the axis of ordinates.

Further, various NMR spectra were determined by using nuclear magnetic resonance (NMR) spectrum analyzer (manufactured by Bruker) and analyzed for its structure. Signals in various NMR spectra are shown below:

¹H-NMR: σ
0.848, 0.860, 0.871,1.236, 1.265, 1.277, 1.289, 1.354, 1.368, 1.507, 1.531, 1.584, 1.923, 1.934, 1.945, 1.964, 1.974, 2.018, 2.029, 2.041, 2.053, 2.208, 2.350, 2.462, 2.575, 2.957, 2.969, 3.728, 3.747, 3.770, 3.777, 3.783, 3.789, 3.971, 3.976, 3.989, 3.994, 5.321, 5.330, 5.346, 5.356, 5.368, 5.378, 5.394, 5.491, 5.501, 5.515, 5.527, 5.539, 5.605, 5.616, 5.629, 5.642, 5.653, 6.455, 6.468.
In ¹H-NMR, the sample was dissolved in deuterated chloroform, and the chemical shift value of residual proton in deuterated chloroform was expressed as 7.24 ppm. Fig. 8 shows ¹H-NMR spectrum. In Fig. 8, the chemical shift value is shown on the axis of abscissas and the signal intensity on the axis of ordinates.

¹³C-NMR: σ
14.10, 22.68, 25.42, 29.20, 29.22, 29.34, 29.36, 29.49, 29.54, 29.62, 29.63, 29.65, 29.69, 31.91, 31.92, 32.42, 32.57, 32.60, 34.36, 40.56, 53.96, 62.45, 74.06, 122.17, 129.72, 130.88, 136.81, 171.83.
In ¹³C-NMR, the sample was dissolved in deuterated chloroform, and the chemical shift value of residual proton in deuterated chloroform was expressed as 77.0 ppm. Fig. 9 shows ¹³C-NMR spectrum. In Fig. 9, the chemical shift value is shown on the axis of abscissas and the signal intensity on the axis of ordinates.

From the above results, the compound represented by the following formula (1) includes one example of compounds that can be estimated as ceramidase inhibitor.

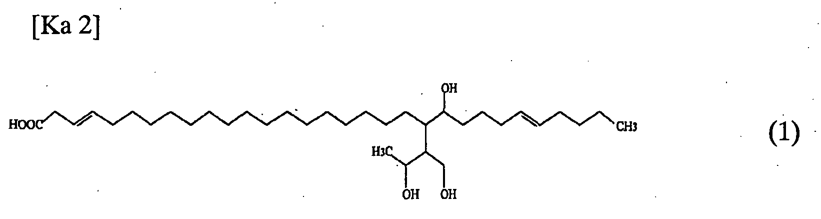

When the ceramidase-inhibiting activity of this purified substance was determined according to the method of Reference Example 1, this substance at a low concentration of 5 µM inhibited 50% of the activity of neutral/alkaline ceramidase derived from a rat brain. On the other hand, inhibition by D-e-MAPP which is commercially available as a conventional ceramidase inhibitor was 28% even at a concentration of 5 mM.

### INDUSTRIAL APPLICABILITY

According to the present invention, there are provided a ceramidase activity inhibitor and a medicament, a quasi-drug, cosmetics and a food, each comprising the ceramidase activity inhibitor.

## Claims

1. A ceramidase activity inhibitor **characterized in that** the inhibitor comprises, as an active ingredient, a processed product derived from at least one plant selected from the group consisting of plants belonging to Ginkgoaceae, plants belonging to Cucurbitaceae, plants belonging to Rutaceae, plants belonging to Laminariaceae, plants belonging to Myrtaceae and plants belonging to Compositae.

2. The ceramidase activity inhibitor according to claim 1, wherein the plant belonging to Ginkgoaceae is ginkgo (*Ginkgo biloba*); the plant belonging to Cucurbitaceae is at least one member selected from the group consisting of Oriental pickling melon (*Cucumis melo* L. var. *conomon* Makino), cucumber (*Cucumis sativus* L.), wax gourd (*Benincasa cerifera* Savi) and bitter cucumber (*Momordica charantia* L.); the plant belonging to Rutaceae is at least one member selected from the group consisting of orange (*Citrus sinensis, Citrus aurantium* or *Citrus reticulate*)*,* grapefruit (*Citrus Paradisi*) and lime (*Citrus aurantifolia*); the plant belonging to Laminariaceae is at least one member selected from the group consisting of gagome (*Kjellmaniella crassifolia* Miyabe), kelp (*Laminaria japonica* Areschoug) and *wakame* seaweed (*Undaria pinnatifida*); the plant belonging to Myrtaceae is eucalyptus; and the plant belonging to Compositae is mugwort (*Artemisia vulgaris* L. var *indica* Maxim.).

3. A ceramide level regulator **characterized in that** the regulator comprises the ceramidase activity inhibitor as defined in claim 1 or 2.

4. A medicament **characterized in that** the medicament comprises the ceramidase activity inhibitor as defined in claim 1 or 2.

5. The medicament according to claim 4, wherein the medicament is a skin medicine for external application.

6. The medicament according to claim 4, wherein the medicament is a therapeutic agent or prophylactic agent for a disease requiring suppression of cellular growth.

7. The medicament according to claim 4, wherein the medicament is a therapeutic agent or prophylactic agent for cancer.

8. A quasi-drug **characterized in that** the quasi-drug comprises the ceramidase activity inhibitor as defined in claim 1 or 2.

9. Cosmetics **characterized in that** the cosmetics comprise the ceramidase activity inhibitor as defined in claim 1 or 2.

10. A food **characterized in that** the food comprises the ceramidase activity inhibitor as defined in claim 1 or 2.

11. A compound having the following physicochemical properties, its derivative, or a pharmacologically acceptable salt thereof:
(1) Mass spectrum: m/z 565 (M+H)⁺;
(2) MS/MS analysis: when the above-mentioned (1) is a parent ion, the daughter ion is m/z 547, m/z 338;
(3) ¹H-NMR (deuterated chloroform): δ
0.848, 0.860, 0.871, 1.236, 1.265, 1.277, 1.289,1.354, 1.368, 1.507, 1.531, 1.584, 1.923, 1.934, 1.945, 1.964, 1.974, 2:018, 2.029, 2.041, 2.053, 2.208, 2.350, 2.462, 2.575, 2.957, 2.969, 3.728, 3.747, 3.770, 3.777, 3.783, 3.789, 3.971, 3.976, 3.989, 3.994, 5.321, 5.330, 5.346, 5.356, 5.368, 5.378, 5.394, 5.491, 5.501, 5.515, 5.527, 5.539, 5.605, 5.616, 5.629, 5.642, 5.653, 6.455, 6.468; and
(4) ¹³C-NMR (deuterated chloroform): δ
14.10, 22.68, 25.42, 29.20, 29.22, 29.34, 29.36, 29.49, 29.54, 29.62, 29.63, 29.65, 29.69, 31.91, 31.92, 32.42, 32.57, 32.60, 34.36, 40.56, 53.96, 62.45, 74.06, 122.17, 129.72, 130.88, 136.81, 171.83.

12. A ceramidase activity inhibitor **characterized in that** the inhibitor comprises as an active ingredient, at least one member selected from the group consisting of the compound, the derivative and the salt as defined in claim 11.

13. A ceramide level regulator **characterized in that** the regulator comprises the ceramidase activity inhibitor as defined in claim 12.

14. A medicament **characterized in that** the medicament comprises the ceramidase activity inhibitor as defined in claim 12.

15. The medicament according to claim 14, wherein the medicament is a skin medicine for external application.

16. The medicament according to claim 14, wherein the medicament is a therapeutic agent or prophylactic agent for a disease requiring suppression of cellular growth.

17. The medicament according to claim 14, wherein the medicament is a therapeutic agent or prophylactic agent for cancer.

18. A quasi-drug **characterized in that** the quasi-drug comprises the ceramidase activity inhibitor as defined in claim 12.

19. Cosmetics **characterized in that** the cosmetics comprise the ceramidase activity inhibitor as defined in claim 12.

20. A food **characterized in that** the food comprises the ceramidase activity inhibitor as defined in claim 12.
